# EUROPEAN PATENT SPECIFICATION

(11) **EP 1 534 265 B1**
(45) Date of publication and mention of the grant of the patent: **22.11.2006**
(21) Application number: 03766348.1
(22) Date of filing: 29.07.2003
(51) Int. Cl.: A61K 31/35, A61K 45/00

(54) **COMBINATION OF AN AROMATASE INHIBITOR WITH A BISPHOSPHONATE**
KOMBINATION VON EINEM AROMATASEHEMMER MIT EINEM BISPHOSPHONAT
COMBINAISON D'UN INHIBITEUR DE L'AROMATASE ET D'UN BIPHOSPHONATE

(30) Priority: 30.07.2002 GB 0217636; 12.11.2002 US 425482 P
(43) Date of publication of application: 01.06.2005
(73) Proprietor: Novartis AG, 4056 Basel (CH); Novartis Pharma GmbH, 1230 Wien (AT)
(72) Inventor: BHATNAGAR, Ajay, S., CH-4132 Muttenz (CH); EVANS, Dean, Brent, CH-4104 Oberwil (CH); GASSER, Jürg, Andreas, CH-4104 Oberwil (CH); GREEN, Jonathan, CH-4144 Arlesheim (CH)
(74) Representative: Crawley, Patrick Edward
(86) International application number: PCT/EP2003/008377
(87) International publication number: WO 2004/012728

(56) References cited:
- FREYER ET AL: "Palliative hormone therapy, low dose chemotherapy and bisphosphonate in breast cancer patients with bone marrow involvement and pancytopenia; report of pilot experience." EUROPEAN J., INTERNAL MEDICINE, vol. 11, 2000, pages 329-333, XP002257266
- LIERSCH ET AL: "Fernmetastasen Chirurgie beim Mammakarzinon." VISZERALCHIRURGIE, vol. 37, April 2002 (2002-04), pages 157-165, XP009018666
- POWLES: "Breast Cancer chemoprevention" THE BREAST, vol. 10, 2001, pages 58-61, XP009018499
- HARVEY: "Issues concerning the role of chemotherapy and hormonal therapy of bone metastases from breast carcinoma" CANCER (SUPPL), vol. 80, 1997, pages 1646-1651, XP002257267
- CONTE ET AL: "Delay in progression of bone metastases in breast cancer patients treated with intravenous palmidronate" J.CLIN.ONCOL., vol. 14, no. 9, 1996, pages 2552-2559, XP009018794
- NEVILLE-WEBBE ET AL: "Zoledronic acid and letrozole induce synergistic levels of apoptosis in breast cancer cell." BONE, vol. 38, 2006, pages S58-S59,

## Description

The present invention provides a combination for the treatment of a disease or condition which responds to aromatase inhibition, in particular a proliferative disease, especially a malignant disease such as breast cancer or similar soft tissue endocrine-sensitive cancer, most preferably breast cancer, comprising an aromatase inhibitor and a N-bisphosphonate for simultaneous, concurrent, separate or sequential use in the prevention of bone loss which is caused by the treatment with an aromatase inhibitor. Also provided is a method of treating a patient suffering from a disease or condition which responds to aromatase inhibition comprising administering to the patient an effective amount of a bisphosphonate and an effective amount of an aromatase inhibitor.

Bisphosphonates are widely used to inhibit osteoclast activity in a variety of both benign and malignant diseases, which involve excessive or inappropriate bone resorption. These pyrophosphate analogs not only reduce the occurrence of skeletal related events but they also provide patients with clinical benefit and improve survival. Bisphosphonates are able to prevent bone resorption *in vivo;* the therapeutic efficacy of bisphosphonates has been demonstrated in the treatment of osteoporosis, osteopenia, Paget's disease of bone, tumour-induced hypercalcemia (TIH) and, more recently, bone metastases (BM) and multiple myeloma (MM) (for review see Fleisch H 1997 Bisphosphonates clinical. In Bisphosphonates in Bone Disease, From the Laboratory to the Patient. Eds: The Parthenon Publishing Group, New York/London pp 68-163). The mechanisms by which bisphosphonates inhibit bone resorption are still not completely understood and seem to vary according to the bisphosphonates studied. Bisphosphonates have been shown to bind strongly to the hydroxyapatite crystals of bone, to reduce bone turn-over and resorption, to decrease the levels of hydroxyproline or alkaline phosphatase in the blood, and in addition to inhibit the formation, recruitment, activation and the activity of osteoclasts.

Aromatase inhibitors have well-known valuable pharmacological properties. They are useful for the inhibition of estrogen biosynthesis in mammals and the treatment or prevention of estrogen dependent disorders responsive thereto, such as mammary tumors (breast carcinoma), endometriosis, premature labor and endometrial tumors in females, as well as gynecomastia in males.

It has now been found that surprisingly the administration of a bisphosphonate, zoledronic acid, to rats treated with an aromatase inhibitor, letrozole, offers long term protection against bone loss in the rats.

Accordingly the present invention provides a combination, such as a combined preparation or a pharmaceutical composition, which comprises (a) an aromatase inhibitor and (b) a N-bisphosphonate in which the active ingredients (a) and (b) are present in each case in free form or in the form of a pharmaceutically acceptable salt, for simultaneous, concurrent, separate or sequential use in the prevention of bone loss which is caused by the treatment with an aromatase inhibitor of a disease or condition which responds to aromatase inhibition, in particular a proliferative disease, especially a malignant disease.

The term "a combined preparation" defines especially a "kit of parts" in the sense that the combination partners (a) and (b) as defined above can be dosed independently or by use of different fixed combinations with distinguished amounts of the combination partners (a) and (b), i.e., simultaneously, concurrently, separately or sequentially. The parts of the kit of parts can then, e.g., be administered simultaneously or chronologically staggered, that is at different time points and with equal or different time intervals for any part of the kit of parts. The ratio of the total amounts of the combination partner (a) to the combination partner (b) to be administered in the combined preparation can be varied, e.g. in order to cope with the needs of a patient sub-population to be treated or the needs of the single patient which different needs can be due to the particular disease, severity of the disease, age, sex, body weight, etc. of the patients.

Further the invention provides the use of an aromatase inhibitor for the preparation of a medicament, for use in combination with a bisphosphonate for treatment of a disease or condition which responds to aromatase inhibition, in particular a proliferative disease, especially a malignant disease, especially with the proviso that the disease or condition is not additionally treated with a chemotherapeutic agent.

In the context of the present application the term "chemotherapeutic agent" refers to antiproliferative agents, such as cytotoxic drugs, but does not embrace hormone therapy drugs such as aromatase inhibitors, tamoxifen or luteinizing hormone-releasing hormone (LH-RH) agonists.

The invention also provides the use of an aromatase inhibitor for the preparation of a medicament, for use in combination with a N-bisphosphonate for prevention of bone loss which is caused by the treatment with an aromatase inhibitor of a disease or condition which responds to aromatase inhibition, in particular a proliferative disease, especially a malignant disease.

In the alternative the invention provides the use of a N-bisphosphonate for the preparation of a medicament, for use in combination with an aromatase inhibitor for treatment of a disease or condition which responds to aromatase inhibition, in particular a proliferative disease, especially a malignant disease, especially with the proviso that the disease or condition is not additionally treated with a chemotherapeutic agent.

The invention also provides the use of a N-bisphosphonate for the preparation of a medicament, for use in combination with an aromatase inhibitor for prevention of bone loss which is caused by the treatment with an aromatase inhibitor of a disease or condition which responds to aromatase inhibition, in particular a proliferative disease, especially a malignant disease.

In yet further aspects the invention provides:
(i) A package comprising a N-bisphosphonate together with instructions for use in combination with an aromatase inhibitor for treatment of a disease or condition which responds to aromatase inhibition, in particular a proliferative disease, especially a malignant disease, especially with the proviso that the disease or condition is not additionally treated with a chemotherapeutic agent, or
(ii) a package comprising an aromatase inhibitor together with instructions for use in combination with a bisphosphonate for treatment of a disease or condition which responds to aromatase inhibition, in particular a proliferative disease, especially a malignant disease, especially with the proviso that the disease or condition is not additionally treated with a chemotherapeutic agent.

In yet another aspect the invention provides:
(i) A package comprising a N-bisphosphonate together with instructions for use in combination with an aromatase inhibitor for prevention of bone loss which is caused by the treatment with an aromatase inhibitor of a disease or condition which responds to aromatase inhibition, in particular a proliferative disease, especially a malignant disease, or
(ii) a package comprising an aromatase inhibitor together with instructions for use in combination with a N-bisphosphonate for prevention of bone loss which is caused by the treatment with an aromatase inhibitor of a disease or condition which responds to aromatase inhibition, in particular a proliferative disease, especially a malignant disease.

Diseases and conditions which may be treated in accordance with the present invention include any of those diseases/conditions which may be treated using aromatase inhibitors, i.e. "a disease or condition which responds to aromatase inhibition", especially those mentioned hereinbefore and hereinafter, including malignant diseases such as in particular endocrine-dependent breast cancer, like for example hormone receptor positive or hormone receptor unknown (advanced or metastatic) breast cancer, e.g. in pre- or post-menopausal women, wherein in pre-menopausal breast cancer an LH-RH agonist such as goserelin is normally given in addition to the aromatase inhibitor.
In particular the present invention may be used in the treatment of diseases and conditions in which treatment with an aromatase inhibitor may induce estrogen deprivation, leading to abnormally increased bone turnover or bone loss. In a very preferred embodiment of the present invention, a combination of an aromatase inhibitor with a bisphosphonate is used in the adjuvant therapy of breast cancer, especially in postmenopausal women; for example, in the treatment of estrogen receptor positive (ER+) and/or progesterone receptor positive (PR+) breast cancer in postmenopausal women. Use of bisphosphonate in combination with an aromatase inhibitor advantageously exerts a bone protective effect, and conveniently may permit treatment with higher doses of aromatase inhibitor, or more prolonged treatment with an aromatase inhibitor than would be possible in the absence ofbisphosphonate.
Thus in further embodiments the invention includes:
- use of a N-bisphosphonate to inhibit bone loss during treatment with an aromatase inhibitor;
- use of a N-bisphosphonate in the manufacture of a medicament for inhibiting bone loss during treatment with an aromatase inhibitor;
- a method of preventing bone loss in a patient suffering from an estrogen dependent disorder and receiving an aromatase inhibitor comprising administering to said patient an effective amount of a bisphosphonate;
- a method of treating of a disease or condition which responds to aromatase inhibition, in particular a proliferative disease, especially a malignant disease, comprising administering an effective amount of an aromatase inhibitor together with an amount of a bisphosphonate effective to inhibit bone loss due to the treatment with an aromatase inhibitor.

In a particularly preferred embodiment the invention provides a method of preventing cancer treatment-related bone loss in a postmenopausal woman with ER+ and/or PR+ breast cancer receiving an aromatase inhibitor as adjuvant therapy comprising administering to said postmenopausal woman an effective amount of a bisphosphonate.

Thus in the present description the terms "treatment" or "treat" refer to both prophylactic or preventative treatment as well as curative or disease modifying treatment, including treatment of patients at risk of contracting the disease or suspected to have contracted the disease as well as patients who are ill or have been diagnosed as suffering from a disease or medical condition.

For the purposes of the present description an N-bisphosphonate is a compound which in addition to the characteristic geminal bisphosphate (P-C-P) moiety comprises a nitrogen containing side chain, e.g. a compound of formula I 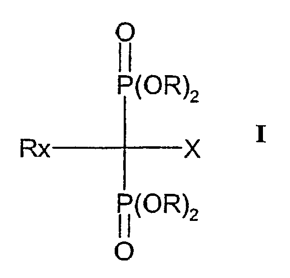 wherein
X is hydrogen, hydroxyl, amino, alkanoyl, or an amino group substituted by C₁-C₄ alkyl, or alkanoyl;
R is hydrogen or C₁-C₄ alkyl and
Rx is a side chain which contains an optionally substituted amino group, or a nitrogen containing heterocycle (including aromatic nitrogen-containing heterocycles),
and pharmaceutically acceptable salts thereof or any hydrate thereof.

Thus, for example, suitable N-bisphosphonates for use in the invention may include the following compounds or a pharmaceutically acceptable salt thereof, or any hydrate thereof: 3-amino-1-hydroxypropane-1,1-diphosphonic acid (pamidronic acid), e.g. pamidronate (APD); 3-(N,N-dimethylamino)-1-hydroxypropane-1,1-diphosphonic acid, e.g. dimethyl-APD; 4-amino-1-hydroxybutane-1,1-diphosphonic acid (alendronic acid), e.g. alendronate; 1-hydroxy-3-(methylpentylamino)-propylidene-bisphosphonic acid, ibandronic acid, e.g. ibandronate; 6-amino-1-hydroxyhexane-1,1-diphosphonic acid; 3-(N-methyl-N-n-pentylamino)-1-hydroxypropane-1,1-diphosphonic acid, e.g. methyl-pentyl-APD (= BM 21.0955); 1-hydroxy-2-(imidazol-1-yl)ethane-1,1-diphosphonic acid, e.g. zoledronic acid; 1-hydroxy-2-(3-pyridyl)ethane-1,1-diphosphonic acid (risedronic acid), e.g. risedronate, including N-methyl pyridinium salts thereof, for example N-methyl pyridinium iodides such as NE-10244 or NE-10446; 3-[N-(2-phenylthioethyl)-N-methylamino]-1-hydroxypropane-1,1-diphosphonic acid; 1-hydroxy-3-(pyrrolidin-1-yl)propane-1,1-diphosphonic acid, e.g. EB 1053 (Leo); 1-(N-phenylaminothiocarbonyl)methane-1,1-diphosphonic acid, e.g. FR 78844 (Fujisawa); 5-benzoyl-3,4-dihydro-2H-pyrazole-3,3-diphosphonic acid tetraethyl ester, e.g. U-81581 (Upjohn); and 1-hydroxy-2-(imidazo[1,2-a]pyridin-3-yl)ethane-1,1-diphosphonic acid, e.g. YM 529.

In one embodiment a particularly preferred N-bisphosphonate for use in the invention comprises a compound of Formula II 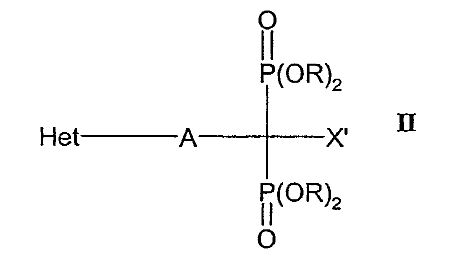 wherein
Het is an imidazole, oxazole, isoxazole, oxadiazole, thiazole, thiadiazole, pyridine, 1,2,3-triazole, 1,2,4-triazole or benzimidazole radical, which is optionally substituted by alkyl, alkoxy, halogen, hydroxyl, carboxyl, an amino group optionally substituted by alkyl or alkanoyl radicals or a benzyl radical optionally substituted by alkyl, nitro, amino or aminoalkyl;
A is a straight-chained or branched, saturated or unsaturated hydrocarbon moiety containing from 1 to 8 carbon atoms;
X' is a hydrogen atom, optionally substituted by alkanoyl, or an amino group optionally substituted by alkyl or alkanoyl radicals, and
R is a hydrogen atom or an alkyl radical,
and the pharmaceutically acceptable salts thereof.

In a further embodiment a particularly preferred bisphosphonate for use in the invention comprises a compound of Formula III 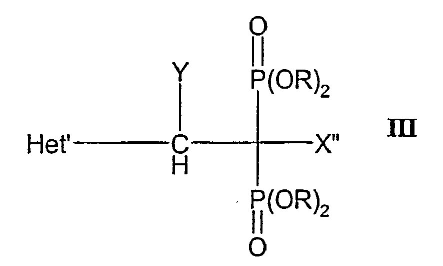 wherein
Het' is a substituted or unsubstituted heteroaromatic five-membered ring selected from the group consisting of imidazolyl, imidazolinyl, isoxazolyl, oxazolyl, oxazolinyl, thiazolyl, thiazolinyl, triazolyl, oxadiazolyl and thiadiazolyl wherein said ring can be partly hydrogenated and wherein said substituents are selected from at least one of the group consisting of C₁-C₄ alkyl, C₁-C₄ alkoxy, phenyl, cyclohexyl, cyclohexylmethyl, halogen and amino and wherein two adjacent alkyl substituents of Het can together form a second ring;
Y is hydrogen or C₁-C₄ alkyl;
X" is hydrogen, hydroxyl, amino, or an amino group substituted by C₁-C₄ alkyl, and
R is hydrogen or C₁-C₄ alkyl;
as well as the pharmaceutically acceptable salts and isomers thereof.

In a yet further embodiment a particularly preferred bisphosphonate for use in the invention comprises a compound of Formula IV 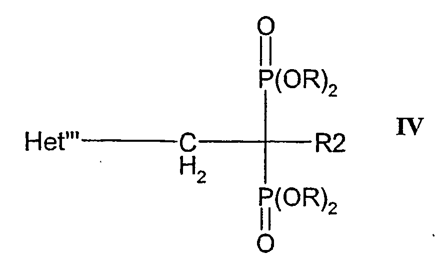 wherein
Het"' is an imidazolyl, 2H-1,2,3-, 1H-1,2,4- or 4H-1,2,4-triazolyl, tetrazolyl, oxazolyl, isoxazolyl, oxadiazolyl, thiazolyl or thiadiazolyl radical which is unsubstituted or C-mono-or di-substituted by lower alkyl, by lower alkoxy, by phenyl which may in turn be mono- or disubstituted by lower alkyl, lower alkoxy and/or halogen, by hydroxy, by di-lower alkylamino, by lower alkylthio and/or by halogen and is N-substituted at a substitutable N-atom by lower alkyl or by phenyl-lower alkyl which may in turn be mono- or di-substituted in the phenyl moiety by lower alkyl, lower alkoxy and/or halogen, and
R2 is hydrogen, hydroxy, amino, lower alkylthio or halogen,
lower radicals having up to and including 7 C-atoms,
or a pharmaceutically acceptable salt thereof.

Examples of particularly preferred N-bisphosphonates for use in the invention are:
2-(1-Methylimidazol-2-yl)-1-hydroxyethane-1,1-diphosphonic acid;
2-(1-Benzylimidazol-2-yl)-1-hydroxyethane-1,1-diphosphonic acid;
2-(1-Methylimidazol-4-yl)-1-hydroxyethane-1,1-diphosphonic acid;
1-Amino-2-(1-methylimidazol-4-yl)ethane-1,1-diphosphonic acid;
1- Amino-2-(1-benzylimidazol-4-yl)ethane-1,1-diphosphonic acid;
2-(1-Methylimidazol-2-yl)ethane-1,1-diphosphonic acid;
2-(1-Benzylimidazol-2-yl)ethane-1,1-diphosphonic acid;
2-(Imidazol-1-yl)-1-hydroxyethane-1,1-diphosphonic acid;
2-(Imidazol-1-yl)ethane-1,1-diphosphonic acid;
2-(4H-1,2,4-triazol-4-yl)-1-hydroxyethane-1,1-diphosphonic acid;
2-(Thiazol-2-yl)ethane-1,1-diphosphonic acid;
2-(Imidazol-2-yl)ethane-1,1-diphosphonic acid;
2-(2-Methylimidazol-4(5)-yl)ethane-1,1-diphosphonic acid;
2-(2-Phenylimidazol-4(5)-yl)ethane-1,1-diphosphonic acid;
2-(4,5-Dimethylimidazol-1-yl)-1-hydroxyethane-1,1-diphosphonic acid; and
2-(2-Methylimidazol-4(5)-yl)-1-hydroxyethane-1,1-diphosphonic acid;
and pharmaceutically acceptable salts thereof.

The most preferred N-bisphosphonate for use in the invention is 2-(imidazol-lyl)-1-hydroxyethane-1,1-diphosphonic acid (zoledronic acid) or a pharmaceutically acceptable salt thereof.

All the N-bisphosphonic acid derivatives mentioned above are well known from the literature. This includes their manufacture (see e.g. EP-A-513760, pp. 13-48). For example, 3-amino-1-hydroxypropane-1,1-diphosphonic acid is prepared as described e.g. in US patent 3,962,432 as well as the disodium salt as in US patents 4,639,338 and 4,711,880, and 1-hydroxy-2-(imidazol-1-yl)ethane-1,1-diphosphonic acid is prepared as described e.g. in US patent 4,939,130. See also US patents 4,777,163 and 4,687,767.

The N-bisphosphonates may be used in the form of an isomer or of a mixture of isomers where appropriate, typically as optical isomers such as enantiomers or diastereoisomers or geometric isomers, typically cis-trans isomers. The optical isomers are obtained in the form of the pure antipodes and/or as racemates.

The N-bisphosphonates can also be used in the form of their hydrates or include other solvents used for their crystallisation.

Suitable aromatase inhibitors for use in the invention may include the following compounds or derivatives thereof or pharmaceutically acceptable salts thereof, or any hydrate or solvate thereof: steroids, especially exemestane and formestane and, in particular, non-steroids, especially aminoglutethimide, vorozole, fadrozole, anastrozole and, very especially, letrozole. Further suitable aromatase inhibitors for use in the present invention are roglethimide, pyridoglutethimide, trilostane, testolactone, atamestane, 1-methyl-1,4-androstadiene-3,17-dione, ketokonazole (see also Cancer-Treat-Res.: 94, 231-254, 1998 and WO 99/30708) and YM511 (K.M. Susaki et al. J. Steroid. Biochem. Molec. Biol. 58, 189-194, 1996) or derivatives thereof or pharmaceutically acceptable salts thereof, or any hydrate or solvate thereof. Preferably, an aromatase inhibitor is selected from exemestane, formestane, aminoglutethimide, fadrozole, anastrozole and letrozole. Exemestane can be administered, e.g., in the form as it is marketed, e.g. under the trademark AROMASIN^{™}. Formestane can be administered, e.g., in the form as it is marketed, e.g. under the trademark LENTARON^{™}. Fadrozole can be administered, e.g., in the form as it is marketed, e.g. under the trademark AFEMA^{™}. Anastrozole can be administered, e.g., in the form as it is marketed, e.g. under the trademark ARIMIDEX^{™}. Letrozole can be administered, e.g., in the form as it is marketed, e.g. under the trademark FEMARA^{™} or FEMAR^{™}. Aminoglutethimide can be administered, e.g., in the form as it is marketed, e.g. under the trademark ORIMETEN^{™}

Furthermore, the structure of the active agents mentioned herein by name may be taken from the actual edition of the standard compendium "The Merck Index" or from databases, e.g. Patents International (e.g. IMS World Publications). The corresponding content thereof is hereby incorporated by reference. Any person skilled in the art is fully enabled, based on these references, to manufacture and test the pharmaceutical indications and properties in standard test models/methods, both *in vitro* and *in vivo.*

Standard test methods to measure aromatase inhibitory activity of a compound *in vitro* and *in vivo* are well known in the art [see e.g.: J. Biol. Chem. 249, 5364 (1974); J. Enzyme Inhib. 4, 169 (1990) and J. Enzyme Inhib. 4, 179 (1990)].

The following compounds and groups of compounds listed below under (a) to (z) and (aa) to (ae) represent further aromatase inhibitors. Each individual group forms a group of aromatase inhibitors that can be used in accordance with the present invention.
(a) The compounds of formulae I and I* as defined in EP-A-165 904. These are especially the compounds of formula I 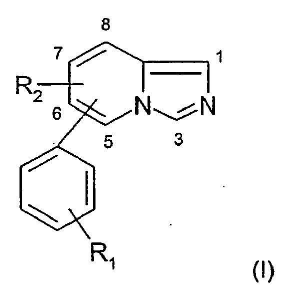 wherein R₁ is hydrogen, lower alkyl; lower alkyl substituted by hydroxy, lower alkoxy, lower alkanoyloxy, lower alkanoyl, amino, lower alkylamino, di-lower alkylamino, halogen, sulfo, carboxy, lower alkoxycarbonyl, carbamoyl or by cyano; nitro, halogen, hydroxy, lower alkoxy, lower alkanoyloxy, phenylsulfonyloxy, lower alkylsulfonyloxy, mercapto, lower alkylthio, lower alkylsulfinyl, lower alkylsulfonyl, lower alkanoylthio, amino, lower alkylamino, di-lower alkylamino, lower alkyleneamino, N-morpholino, N-thiomorpholino, N-piperazino that is unsubstituted or lower alkyl-substituted in the 4-position, tri-lower alkylammonio, sulfo, lower alkoxysulfonyl, sulfamoyl, lower alkylsulfamoyl, di-lower alkylsulfamoyl, formyl; iminomethyl that is unsubstituted or substituted at the nitrogen atom by hydroxy, lower alkoxy, lower alkanoyloxy, lower alkyl, phenyl or by amino; C₂-C₇alkanoyl, benzoyl, carboxy, lower alkoxycarbonyl, carbamoyl, lower alkylcarbamoyl, di-lower alkylcarbamoyl, cyano, 5-tetrazolyl, unsubstituted or lower alkyl-substituted 4,5-dihydro-2-oxazolyl or hydroxycarbamoyl; and R₂ is hydrogen, lower alkyl, phenyl-lower alkyl, carboxy-lower alkyl, lower alkoxycarbonyl-lower alkyl, halogen, hydroxy, lower alkoxy, lower alkanoyloxy, mercapto, lower alkylthio, phenyl-lower alkylthio, phenylthio, lower alkanoylthio, carboxy, lower alkoxycarbonyl or lower alkanoyl; the 7,8-dihydro derivatives thereof; and the compounds of formula I* 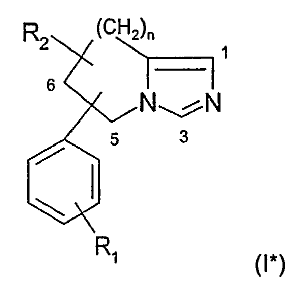 wherein n is 0, 1, 2, 3 or 4; and R₁ and R₂ are as defined above for formula I; it being possible for the phenyl ring in the radicals phenylsulfonyloxy, phenyliminomethyl, benzoyl, phenyl-lower alkyl, phenyl-lower alkylthio and phenylthio to be unsubstituted or substituted by lower alkyl, lower alkoxy or by halogen; it being possible in a compound of formula I* for the two substituents C₆H₄-R₁ and R₂ to be linked to each of the saturated carbon atoms of the saturated ring, either both to the same carbon atom or both to different carbon atoms, and pharmaceutically acceptable salts thereof.
   Preferred compounds of this group are:
   (1) 5-(p-cyanophenyl)imidazo[1,5-a]pyridine,
   (2) 5-(p-ethoxycarbonylphenyl)imidazo[1,5-a]pyridine,
   (3) 5-(p-carboxyphenyl)imidazo[1,5-a]pyridine,
   (4) 5-(p-tert-butylaminocarbonylphenyl)imidazo[1,5-a]pyridine,
   (5) 5-(p-ethoxycarbonylphenyl)-5,6,7,8-tetrahydroimidazo[1,5-a]pyridine,
   (6) 5-(p-carboxyphenyl)-5,6,7,8-tetrahydroimidazo[1,5-a]pyridine,
   (7) 5-(p-carbamoylphenyl)-5,6,7,8-tetrahydroimidazo[1,5-a]pyridine,
   (8) 5-(p-tolyl)-5,6,7,8-tetrahydroimidazo[1,5-a]pyridine,
   (9) 5-(p-hydroxymethylphenyl)imidazo[1,5-a]pyridine,
   (10) 5-(p-cyanophenyl)-7,8-dihydroimidazo[1,5-a]pyridine,
   (11) 5-(p-bromophenyl)-5,6,7,8-tetrahydroimidazo[1,5-a]pyridine,
   (12) 5-(p-hydroxymethylphenyl)-5,6,7,8-tetrahydroimidazo[1,5-a]pyridine,
   (13) 5-(p-formylphenyl)-5,6,7,8-tetrahydroimidazo[1,5-a]pyridine,
   (14) 5-(p-cyanophenyl)-5-methylthio-5,6,7,8-tetrahydroimidazo[1,5-a]pyridine,
   (15) 5-(p-cyanophenyl)-5-ethoxycarbonyl-5,6,7,8-tetrahydroimidazo[1,5-a]pyridine,
   (16) 5-(p-aminophenyl)-5,6,7,8-tetrahydroimidazo[1,5-a]pyridine,
   (17) 5-(p-formylphenyl)imidazo[1,5-a]pyridine,
   (18) 5-(p-carbamoylphenyl)imidazo[1,5-a]pyridine,
   (19) 5H-5-(4-tert-butylaminocarbonylphenyl)-6,7-dihydropyrrolo[1 ,2-c]imidazole,
   (20) 5H-5-(4-cyanophenyl)-6,7-dihydropyrrolo[1,2-c]imidazole,
   (21) 5H-5-(4-cyanophenyl)-6,7,8,9-tetrahydroimidazo[1,5-a]azepine,
   (22) 5-(4-cyanophenyl)-6-ethoxycarbonylmethyl-5,6,7,8-tetrahydroimidazo[1,5-a]pyridine,
   (23) 5-(4-cyanophenyl)-6-carboxymethyl-5,6,7,8-tetrahydroimidazo[1,5-a]pyridine,
   (24) 5-benzyl-5-(4-cyanophenyl)-5,6,7,8-tetrahydroimidazo[1,5-a]pyridine,
   (25) 7-(p-cyanophenyl)-5,6,7,8-tetrahydroimidazo[1,5-a]pyridine,
   (26) 7-(p-carbamoylphenyl)-5,6,7,8-tetrahydroimidazo[1,5-a]pyridine,
   (27) 5-(p-cyanophenyl)-5,6,7,8-tetrahydroimidazo[1,5-a]pyridine (=fadrozole).
(b) The compounds of formula I as defined in EP-A 236 940. These are especially the compounds of formula I 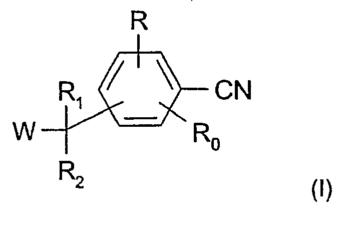 wherein R and R₀, independently of one another, are each hydrogen or lower alkyl, or R and R₀ at adjacent carbon atoms, together with the benzene ring to which they are bonded, form a naphthalene or tetrahydronaphthalene ring; wherein R₁ is hydrogen, lower alkyl, aryl, aryl-lower alkyl or lower alkenyl; R₂ is hydrogen, lower alkyl, aryl, aryl-lower alkyl, (lower alkyl, aryl or aryl-lower alkyl)-thio or lower alkenyl, or wherein R₁ and R₂ together are lower alkylidene or C₄-C₆alkylene; wherein W is 1-imidazolyl, 1-(1,2,4 or 1,3,4 )-triazolyl, 3-pyridyl or one of the mentioned heterocyclic radicals substituted by lower alkyl; and aryl within the context of the above definitions has the following meanings: phenyl that is unsubstituted or substituted by one or two substituents from the group lower alkyl, lower alkoxy, hydroxy, lower alkanoyloxy, nitro, amino, halogen, trifluoromethyl, cyano, carboxy, lower alkoxycarbonyl, carbamoyl, N-lower alkylcarbamoyl, N,N-di-lower alkylcarbamoyl, lower alkanoyl, benzoyl, lower alkylsulfonyl, sulfamoyl, N-lower alkylsulfamoyl and N,N-di-lower alkylsulfamoyl; also thienyl, indolyl, pyridyl or furyl, or one of the four last-mentioned heterocyclic radicals monosubstituted by lower alkyl, lower alkoxy, cyano or by halogen; and pharmaceutically acceptable salts thereof.
   Preferred compounds of this group are:
   (1) 4-[α-(4-cyanophenyl)-1-imidazolylmethyl]-benzonitrile,
   (2) 4-[α-(3-pyridyl)-1-imidazolylmethyl]-benzonitrile,
   (3) 4-[α-(4-cyanobenzyl)-1-imidazolylmethyl]-benzonitrile,
   (4) 1-(4-cyanophenyl)-1-(1-imidazolyl)-ethylene,
   (5) 4-[α-(4-cyanophenyl)-1-(1,2,4-triazolyl)methyl]-benzonitrile (=letrozole),
   (6) 4-[α-(4-cyanophenyl)-3-pyridylmethyl]-benzonitrile.
(c) The compounds of formula I as defined in EP-A-408 509. These are especially the compounds of formula I 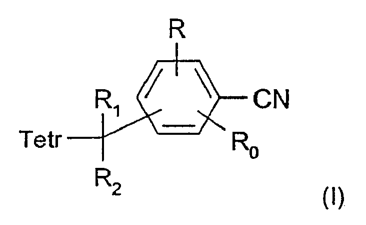 wherein Tetr is 1- or 2-tetrazolyl that is unsubstituted or substituted in the 5-position by lower alkyl, phenyl-lower alkyl or by lower alkanoyl; R₁ and R₂, independently of one another, are each hydrogen; lower alkyl that is unsubstituted or substituted by hydroxy, lower alkoxy, halogen, carboxy, lower alkoxycarbonyl, (amino, lower alkylamino or di-lower alkylamino)-carbonyl or by cyano; lower alkenyl, aryl, heteroaryl, aryl-lower alkyl, C₃-C₆-cycloalkyl, C₃-C₆₋cycloalkyl-lower alkyl, lower alkylthio, arylthio or aryl-lower alkylthio; or R₁ and R₂ together are straight-chained C₄-C₆alkylene that is unsubstituted or substituted by lower alkyl, or are a group -(CH₂)ₘ-1,2-phenylene-(CH₂)ₙ wherein m and n, independently of one another, are each 1 or 2 and 1,2-phenylene is unsubstituted or substituted in the same way as phenyl in the definition of aryl below, or are lower alkylidene that is unsubstituted or mono- or di-substituted by aryl; and R and R₀, independently of one another, are each hydrogen or lower alkyl; or R and R₀ together, located at adjacent carbon atoms of the benzene ring, are a benzo group that is unsubstituted or substituted in the same way as phenyl in the definition of aryl below; aryl in the above definitions being phenyl that is unsubstituted or substituted by one or more substituents from the group consisting of lower alkyl, lower alkoxy, hydroxy, lower alkanoyloxy, nitro, amino, halogen, trifluoromethyl, carboxy, lower alkoxycarbonyl, (amino, lower alkylamino or di-lower alkylamino)-carbonyl, cyano, lower alkanoyl, benzoyl, lower alkylsulfonyl and (amino, lower alkylamino or di-lower alkylamino)-sulfonyl; heteroaryl in the above definitions being an aromatic heterocyclic radical from the group consisting of pyrrolyl, pyrazolyl, imidazolyl, triazolyl, tetrazolyl, furanyl, thienyl, isoxazolyl, oxazolyl, oxadiazolyl, isothiazolyl, thiazolyl, thiadiazolyl, pyridyl, pyridazinyl, pyrimidyl, pyrazinyl, triazinyl, indolyl, isoindolyl, benzimidazolyl, benzotriazolyl, benzofuranyl, benzothienyl, benzoxazolyl, benzothiazolyl, benzoxadiazolyl, benzothiadiazolyl, quinolyl and isoquinolyl that is unsubstituted or substituted in the same way as phenyl in the definition of aryl above; and pharmaceutically acceptable salts thereof.
   Preferred compounds of this group are:
   (1) 4-(2-tetrazolyl)methyl-benzonitrile,
   (2) 4-[α-(4-cyanophenyl)-(2-tetrazolyl)methyl]-benzonitrile,
   (3) 1-cyano-4-(1-tetrazolyl)methyl-naphthalene,
   (4) 4-[(α-(4-cyanophenyl)-(1-tetrazolyl)methyl]-benzonitrile.
(d) The compounds of formula I as defined in European Patent Application No.91810110.6. These are especially the compounds of formula I 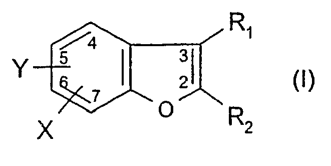 wherein X is halogen, cyano, carbamoyl, N-lower alkylcarbamoyl, N-cycloalkyl-lower alkylcarbamoyl, N,N-di-lower alkylcarbamoyl, N-arylcarbamoyl, hydroxy, lower alkoxy, aryl-lower alkoxy or aryloxy, wherein aryl is phenyl or naphthyl, each of which is unsubstituted or substituted by lower alkyl, hydroxy, lower alkoxy, halogen and/or by trifluoromethyl; Y is a group -CH₂-A wherein A is 1-imidazolyl, 1-(1,2,4-triazolyl), 1-(1,3,4-triazolyl), 1-(1,2,3-triazolyl), 1-(1,2,5-triazolyl), 1-tetrazolyl or 2-tetrazolyl, or Y is hydrogen, R₁ and R₂, independently of one another, are each hydrogen, lower alkyl or a group -CH₂-A as defined for Y, or R₁ and R₂ together are -(CH₂)ₙ- wherein n is 3, 4 or 5, with the proviso that one of the radicals Y, R₁ and R₂ is a group -CH₂-A, with the further proviso that in a group -CH₂-A as a meaning of R₁ or R₂, A is other than 1-imidazolyl when X is bromine, cyano or carbamoyl, and with the proviso that in a group -CH₂-A as a meaning of Y, A is other than 1-imidazolyl when X is halogen or lower alkoxy, R₁ is hydrogen and R₂ is hydrogen or lower alkyl, and pharmaceutically acceptable salts thereof.
   Preferred compounds of this group are:
   (1) 7-cyano-4-[1-(1,2,4-triazolyl)methyl]-2,3-dimethylbenzofuran,
   (2) 7-cyano-4-(1-imidazolylmethyl)-2,3-dimethylbenzofuran,
   (3) 7-carbamoyl-4-(1-imidazolylmethyl)-2,3-dimethylbenzofuran,
   (4) 7-N-(cyclohexylmethyl)carbamoyl-4-(1-imidazolylmethyl)-2,3-dimethylbenzofuran.
(e) The compounds of formula I as defined in Swiss Patent Application 1339/90-7. These are especially the compounds of formula I 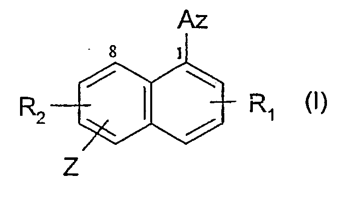 wherein the dotted line denotes an additional bond or no additional bond, Az is imidazolyl, triazolyl or tetrazolyl bonded via a ring nitrogen atom, each of those radicals being unsubstituted or substituted at carbon atoms by lower alkyl or by aryl-lower alkyl, Z is carboxy, lower alkoxycarbonyl, carbamoyl, N-lower alkylcarbamoyl, N,N-di-lower alkylcarbamoyl, N-arylcarbamoyl, cyano, halogen, hydroxy, lower alkoxy, aryl-lower alkoxy, aryloxy, lower alkyl, trifluoromethyl or aryl-lower alkyl, and R₁ and R₂, independently of one another, are each hydrogen, lower alkyl, lower alkoxy, hydroxy, halogen or trifluoromethyl; aryl being phenyl or naphthyl each of which is unsubstituted or substituted by one or two substituents from the group consisting of lower alkyl, lower alkoxy, hydroxy, halogen and trifluoromethyl; with the proviso that neither Z nor R₂ is hydroxy in the 8-position, and pharmaceutically acceptable salts thereof.
   Preferred compounds of this group are:
   (1) 6-cyano-1-(1-imidazolyl)-3,4-dihydronaphthalene,
   (2) 6-cyano-1-[1-(1,2,4-triazolyl)]-3,4-dihydronaphthalene,
   (3) 6-chloro-1-(1-imidazolyl)-3,4-dihydronaphthalene,
   (4) 6-bromo-1-(1-imidazolyl)-3,4-dihydronaphthalene.
(f) The compounds of formula I as defined in Swiss Patent Application 3014/90-0. These are especially the compounds of formula I 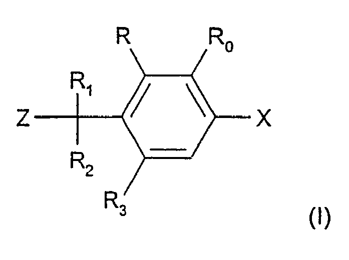 wherein Z is a five-membered nitrogen-containing heteroaromatic ring selected from the group 5-isothiazolyl, 5-thiazolyl, 5-isoxazolyl, 5-oxazolyl, 5-(1,2,3-thiadiazolyl), 5-(1,2,3-oxadiazolyl), 3-(1,2,5-thiadiazolyl), 3-(1 ,2,5-oxadiazolyl), 4-isothiazolyl, 4-isoxazolyl, 4-(1,2,3-thiadiazolyl), 4-(1,2,3-oxadiazolyl), 2-(1,3,4-thiadiazolyl), 2-(1,3,4-oxadiazolyl), 5-(1,2,4-thiadiazolyl) and 5-(1 ,2,4-oxadiazolyl); R and R₀ are hydrogen; or R and R₀ together are a benzo group that is unsubstituted or substituted by lower alkyl, lower alkoxy, hydroxy, halogen or by trifluoromethyl; R₁ is hydrogen, hydroxy, chlorine or fluorine; R₃ is hydrogen; R₂ is hydrogen, lower alkyl or phenyl that is unsubstituted or substituted by lower alkyl, lower alkoxy, hydroxy, halogen, trifluoromethyl or by cyano; or R₁ and R₂ together are methylidene; or R₂ and R₃ together are -(CH₂)₃-; or R₁ and R₂ and R₃ together are a group =CH-(CH₂)₂- wherein the single bond is linked to the benzene ring; X is cyano; and X may also be halogen when R₂ and R₃ together are -(CH₂)₃- or R₁ and R₂ and R₃ together are a group =CH-(CH₂)₂-; and pharmaceutically acceptable salts thereof.
   Preferred compounds of this group are:
   (1) 4-[α-(4-cyanophenyl)-α-hydroxy-5-isothiazolylmethyl]-benzonitrile,
   (2) 4-[α-(4-cyanophenyl)-5-isothiazolylmethyl]-benzonitrile,
   (3) 4-[α-(4-cyanophenyl)-5-thiazolylmethyl]-benzonitrile,
   (4) 1-(4-cyanophenyl)-1-(5-thiazolyl)-ethylene,
   (5) 6-cyano-1-(5-isothiazolyl)-3,4-dihydronaphthalene,
   (6) 6-cyano-1-(5-thiazolyl)-3,4-dihydronaphthalene.
(g) The compounds of formula VI as defined in Swiss Patent Application 3014/90-0. These are especially the compounds of formula VI 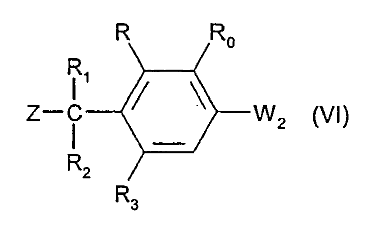 wherein Z is a five-membered nitrogen-containing heteroaromatic ring selected from the group 5-isothiazolyl, 5-thiazolyl, 5-isoxazolyl, 5-oxazolyl, 5-(1,2,3-thiadiazolyl), 5-(1,2,3-oxadiazolyl), 3-(1,2,5-thiadiazolyl), 3-(1,2,5-oxadiazolyl), 4-isothiazolyl, 4-isoxazolyl, 4-(1,2,3-thiadiazolyl), 4-(1,2,3-oxadiazolyl), 2-(1,3,4-thiadiazolyl), 2-(1,3,4-oxadiazolyl), 5-(1,2,4-thiadiazolyl) and 5-(1,2,4-oxadiazolyl); R and R₀ are each hydrogen; or R and R₀ together are a benzo group that is unsubstituted or substituted by lower alkyl, lower alkoxy, hydroxy, halogen or by trifluoromethyl; R₁ is hydrogen, hydroxy, chlorine or fluorine; R₃ is hydrogen; R₂ is hydrogen, lower alkyl or phenyl that is unsubstituted or substituted by lower alkyl, lower alkoxy, hydroxy, halogen, trifluoromethyl, aryl-lower alkoxy or by aryloxy; or R₁ and R₂ together are methylidene, and W₂ is halogen, hydroxy, lower alkoxy, aryl-lower alkoxy or aryloxy; aryl in each case being phenyl that is unsubstituted or substituted by lower alkyl, lower alkoxy, hydroxy, halogen or by trifluoromethyl; and pharmaceutically acceptable salts thereof.
   Preferred compounds of this group are:
   (1) bis(4,4'-bromophenyl)-(5-isothiazolyl)methanol,
   (2) bis(4,4'-bromophenyl)-(5-isothiazolyl)methane,
   (3) bis(4,4'-bromophenyl)-(5-thiazolyl)methanol,
   (4) bis(4,4'-bromophenyl)-(5-thiazolyl)methane.
(h) The compounds of formula I as defined in Swiss Patent Application 3923/90-4. These are especially the compounds of formula I 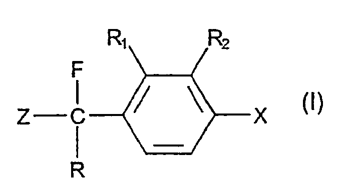 wherein Z is imidazolyl, triazolyl, tetrazolyl, pyrrolyl, pyrazolyl, indolyl, isoindolyl, benzimidazolyl, benzopyrazolyl, benzotriazolyl, pyridyl, pyrimidyl, pyrazinyl, pyridazinyl, triazinyl, quinolinyl or isoquinolinyl, all those radicals being bonded via their heterocyclic rings and all those radicals being unsubstituted or substituted by lower alkyl, hydroxy, lower alkoxy, halogen or by trifluoromethyl; R₁ and R₂, independently of one another, are each hydrogen or lower alkyl; or R₁ and R₂ together are C₃-C₄alkylene, or a benzo group that is unsubstituted or substituted as indicated below for aryl; R is hydrogen, lower alkyl, aryl or heteroaryl, and X is cyano, carbamoyl, N-lower alkylcarbamoyl, N,N-di-lower alkylcarbamoyl, N,N-lower alkylenecarbamoyl; N,N-lower alkylenecarbamoyl interrupted by -O-, -S- or -NR"-, wherein R" is hydrogen, lower alkyl or lower alkanoyl; N-cycloalkylcarbamoyl, N-(lower alkyl-substituted cycloalkyl)-carbamoyl, N-cycloalkyl-lower alkylcarbamoyl, N-(lower alkyl-substituted cycloalkyl)-lower alkylcarbamoyl, N-aryl-lower alkylcarbamoyl, N-arylcarbamoyl, N-hydroxycarbamoyl, hydroxy, lower alkoxy, aryl-lower alkoxy or aryloxy; and wherein X is also halogen when Z is imidazolyl, triazolyl, tetrazolyl, pyrrolyl, pyrazolyl, indolyl, isoindolyl, benzimidazolyl, benzopyrazolyl or benzotriazolyl;
   wherein aryl is phenyl or naphthyl, these radicals being unsubstituted or substituted by from 1 to 4 substituents from the group consisting of lower alkyl, lower alkenyl, lower alkynyl, lower alkylene (linked to two adjacent carbon atoms), C₃-C₈cycloalkyl, phenyl-lower alkyl, phenyl; lower alkyl that is substituted in turn by hydroxy, lower alkoxy, phenyl-lower alkoxy, lower alkanoyloxy, halogen, amino, lower alkylamino, di-lower alkylamino, mercapto, lower alkylthio, lower alkylsulfinyl, lower alkylsulfonyl, carboxy, lower alkoxycarbonyl, carbamoyl, N-lower alkylcarbamoyl, N,N-di-lower alkylcarbamoyl and/or by cyano; hydroxy; lower alkoxy, halo-lower alkoxy, phenyl-lower alkoxy, phenoxy, lower alkenyloxy, halo-lower alkenyloxy, lower alkynyloxy, lower alkylenedioxy (linked to two adjacent carbon atoms), lower alkanoyloxy, phenyl-lower alkanoyloxy, phenylcarbonyloxy, mercapto, lower alkylthio, phenyl-lower alkylthio, phenylthio, lower alkylsulfinyl, phenyl-lower alkylsulfinyl, phenylsulfinyl, lower alkylsulfonyl, phenyl-lower alkylsulfonyl, phenylsulfonyl, halogen, nitro, amino, lower alkylamino, C₃-C₈cycloalkylamino, phenyl-lower alkylamino, phenylamino, di-lower alkylamino, N-lower alkyl-N-phenylamino, N-lower alkyl-N-phenyl-lower alkylamino; lower alkyleneamino or lower alkyleneamino interrupted by -O-, -S- or -NR"- (wherein R" is hydrogen, lower alkyl ur lower alkanoyl); lower alkanoylamino, phenyl-lower alkanoylamino, phenylcarbonylamino, lower alkanoyl, phenyl-lower alkanoyl, phenylcarbonyl, carboxy, lower alkoxycarbonyl, carbamoyl, N-lower alkylcarbamoyl, N,N-di-lower alkylcarbamoyl, N,N-lower alkylenecarbamoyl; N,N-lower alkylenecarbamoyl interrupted by -O-, -S- or -NR"-, wherein R" is hydrogen, lower alkyl or lower alkanoyl; N-cycloalkylcarbamoyl, N-(lower alkyl-substituted cycloalkyl)-carbamoyl, N-cycloalkyl-lower alkylcarbamoyl, N-(lower alkyl-substituted cycloalkyl)-lower alkylcarbamoyl, N-hydroxycarbamoyl, N-phenyl-lower alkylcarbamoyl, N-phenylcarbamoyl, cyano, sulfo, lower alkoxysulfonyl, sulfamoyl, N-lower alkylsulfamoyl, N,N-di-lower alkylsulfamoyl and N-phenylsulfamoyl; the phenyl groups occurring in the substituents of phenyl and naphthyl in turn being unsubstituted or substituted by lower alkyl, lower alkoxy, hydroxy, halogen and/or by trifluoromethyl;
   wherein heteroaryl is indolyl, isoindolyl, benzimidazolyl, benzopyrazolyl, benzotriazolyl, benzo[b]furanyl, benzo[b]thienyl, benzoxazolyl or benzothiazolyl, those radicals being unsubstituted or substituted by from 1 to 3 identical or different substituents selected from lower alkyl, hydroxy, lower alkoxy, halogen, cyano and trifluoromethyl; and pharmaceutically acceptable salts thereof.
   Those compounds are especially the compounds of formula I wherein Z is 1-imidazolyl, 1-(1,2,4-triazolyl), 1-(1,3,4-triazolyl), 1-(1,2,3-triazolyl), 1-tetrazolyl, 2-tetrazolyl, 3-pyridyl, 4-pyridyl, 4-pyrimidyl, 5-pyrimidinyl or 2-pyrazinyl; R₁ and R₂, independently of one another, are each hydrogen or lower alkyl; or R₁ and R₂ together are 1,4-butylene or a benzo group; R is lower alkyl; phenyl that is unsubstituted or substituted by cyano, carbamoyl, halogen, lower alkyl, trifluoromethyl, hydroxy, lower alkoxy or by phenoxy; or benzotriazolyl or benzo[b]-furanyl, the last two radicals being unsubstituted or substituted by from 1 to 3 identical or different substituents selected from lower alkyl, halogen and cyano; and X is cyano or carbamoyl; and wherein X is also halogen when Z is 1-imidazolyl, 1-(1,2,4-triazolyl), 1-(1,3,4-triazolyl), 1-(1,2,3-triazolyl), 1-tetrazolyl or 2-tetrazolyl; and pharmaceutically acceptable salts thereof.
   Preferred compounds of this group are:
   (1) 4-[α-(4-cyanophenyl)-α-fluoro-1-(1,2,4-triazolyl)methyl]-benzonitrile,
   (2) 4-[α-(4-cyanophenyl)-α-fluoro-(2-tetrazolyl)methyl]-benzonitrile,
   (3) 4-[α-(4-cyanophenyl)-α-fluoro-(1-tetrazolyl)methyl]-benzonitrile,
   (4) 4-[α-(4-cyanophenyl)-α-fluoro-(1-imidazolyl)methyl]-benzonitrile,
   (5) 1-methyl-6-[α-(4-chlorophenyl)-α-fluoro-1-(1,2,4-triazolyl)methyl]-benzotriazole,
   (6) 4-[α-(4-cyanophenyl)-α-fluoro-1-(1,2,3-triazolyl)methyl]-benzonitrile,
   (7) 7-cyano-4-[α-(4-cyanophenyl)-α-fluoro-1-(1,2,4-triazolyl)methyl]-2,3-dimethylbenzo[b]-furan,
   (8) 4-[α-(4-bromophenyl)-α-fluoro-1-(1,2,4-triazolyl)methyl]-benzonitrile,
   (9) 4-[α-(4-cyanophenyl)-α-fluoro-(5-pyrimidyl)methyl]-benzonitrile,
   (10) 4-[α-(4-bromophenyl)-α-fluoro-(5-pyrimidyl)methyl]-benzonitrile,
   (11) 4-[α-(4-cyanophenyl)-α-fluoro-(3-pyridyl)methyl]-benzonitrile,
   (12) 7-bromo-4-[α-(4-cyanophenyl)-α-fluoro-(1-imidazolyl)methyl]-2,3-dimethylbenzo[b]furan,
   (13) 7-bromo-4-[α-(4-cyanophenyl)-α-fluoro-1-(1,2,4-triazolyl)methyl]-2,3-dimethylbenzo-[b]furan,
   (14) 4-[α-(4-cyanophenyl)-α-fluoro-(5-pyrimidyl)methyl]-benzonitrile,
   (15) 4-[α-(4-bromophenyl)-α-fluoro-(5-pyrimidyl)methyl]-benzonitrile,
   (16) 4-[α-(4-cyanophenyl)-1-(1,2,3-triazolyl)methyl]-benzonitrile,
   (17) 2,3-dimethyl-4-[α-(4-cyanophenyl)-1-(1,2,4-triazolyl)methyl]-7-cyano-benzo[b]furan,
   (18) 4-[α-(4-cyanophenyl)-(5-pyrimidyl)methyl]-benzonitrile,
   (19) 4-[α-(4-bromophenyl)-(5-pyrimidyl)methyl]-benzonitrile,
   (20) 2,3-dimethyl-4-[α-(4-cyanophenyl)-(1-imidazolyl)methyl]-7-bromo-benzo[b]furan,
   (21) 2,3-dimethyl-4-[α-(4-cyanophenyl)-1-(1,2,4-triazolyl)methyl]-7-bromo-benzo[b]furan.
(i) The compounds of formula I as defined in EP-A-114 033. These are especially the compounds of formula I 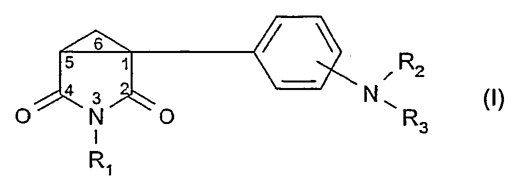 wherein R₁ is hydrogen, R₂ is hydrogen, sulfo, C₁-C₇alkanoyl or C₁-C₇alkanesulfonyl and R₃ is hydrogen, or wherein R₁ is C₁-C₁₂alkyl, C₂-C₁₂alkenyl, C₂-C₇alkynyl, C₃-C₁₀cycloalkyl, C₃₋C₁₀cycloalkenyl, C₃-C₆cycloalkyl-C₁-C₄alkyl, C₃-C₆cycloalkyl-C₂-C₄alkenyl or C₃-C₆cycloalkenyl-C₁-C₄alkyl, R₂ is hydrogen, C₁-C₇alkyl, sulfo, C₁-C₇alkanoyl or C₁-C₇alkanesulfonyl and R₃ is hydrogen or C₁-C₇alkyl, and pharmaceutically acceptable salts of those compounds.
   Preferred compounds of this group are:
   (1) 1-(4-aminophenyl)-3-methyl-3-azabicyclo[3.1.0]hexane-2,4-dione,
   (2) 1-(4-aminophenyl)-3-n-propyl-3-azabicyclo[3.1.0]hexane-2,4-dione,
   (3) 1-(4-aminophenyl)-3-isobutyl-3-azabicyclo[3.1.0]hexane-2,4-dione,
   (4) 1-(4-aminophenyl)-3-n-heptyl-3-azabicydo[3.1.0]hexane-2,4-dione,
   (5) 1-(4-aminophenyl)-3-cyclohexylmethyl-3-azabicyclo[3.1.0]hexane-2,4-dione.
(j) The compounds of formula I as defined in EP-A-166 692. These are especially the compounds of formula I 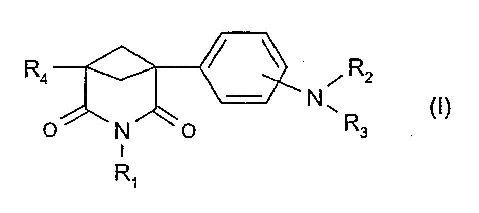 wherein R₁ is hydrogen, alkyl having from 1 to 12 carbon atoms, alkenyl having from 2 to 12 carbon atoms, lower alkynyl, cycloalkyl or cycloalkenyl each having from 3 to 10 carbon atoms, cycloalkyl-lower alkyl having from 4 to 10 carbon atoms, cycloalkyl-lower alkenyl having from 5 to 10 carbon atoms, cycloalkenyl-lower alkyl having from 4 to 10 carbon atoms, or aryl having from 6 to 12 carbon atoms or aryl-lower alkyl having from 7 to 15 carbon atoms, each of which is unsubstituted or substituted by lower alkyl, hydroxy, lower alkoxy, acyloxy, amino, lower alkylamino, di-lower alkylamino, acylamino or by halogen, R₂ is hydrogen, lower alkyl, sulfo, lower alkanoyl or lower alkanesulfonyl, R₃ is hydrogen or lower alkyl and R₄ is hydrogen, lower alkyl, phenyl or phenyl substituted by -N(R₂)(R₃), and pharmaceutically acceptable salts thereof, radicals described as "lower" containing up to and including 7 carbon atoms.
   Preferred compounds of this group are:
   (1) 1-(4-aminophenyl)-3-n-propyl-3-azabicyclo[3.1.1]heptane-2,4-dione,
   (2) 1-(4-aminophenyl)-3-methyl-3-azabicyclo[3.1.1]heptane-2,4-dione,
   (3) 1-(4-aminophenyl)-3-n-decyl-3-azabicyclo[3.1.1]heptane-2,4-dione,
   (4) 1-(4-aminophenyl)-3-cyclohexyl-3-azabicydo[3.1.1]heptane-2,4-dione,
   (5) 1-(4-aminophenyl)-3-cyclohexylmethyl-3-azabicydo[3.1.1]heptane-2,4-dione.
(k) The compounds of formula I as defined in EP-A-356 673. These are especially the compounds of formula I 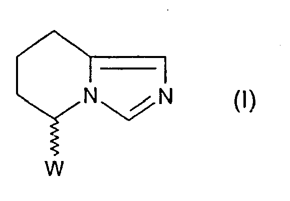 wherein W
   (α) is a 2-naphthyl or 1-anthryl radical, wherein each benzene ring is unsubstituted or substituted by a substituent selected from halogen, hydroxy, carboxy, cyano and nitro; or
   (β) is 4-pyridyl, 2-pyrimidyl or 2-pyrazinyl, each of those radicals being unsubstituted or substituted by a substituent selected from halogen, cyano, nitro, C₁-C₄alkoxy and C₂-C₅alkoxycarbonyl; and pharmaceutically acceptable salts thereof.
   Preferred compounds of this group are:
   (1) 5-(2'-naphthyl)-5,6,7,8-tetrahydroimidazo[1,5-a]pyridine,
   (2) 5-(4'-pyridyl)-5,6,7,8-tetrahydroimidazo[1,5-a]pyridine.
(I) The compounds of formula I or la as defined in EP-A-337 929. These are especially the compounds of formula I/la 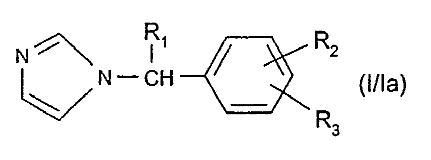 wherein R₁ is hydrogen, methyl, ethyl, propyl, propenyl, isopropyl, butyl, hexyl, octyl, decyl, cyclopentyl, cyclohexyl, cyclopentylmethyl, cyclohexylmethyl or benzyl, R₂ is benzyloxy, 3-bromo-, 4-bromo-, 4-chloro-, 2,3-, 2,4-, 4,5- or 4,6-dichloro-benzyloxy, and R₃ is cyano; C₂₋C₁₀alkanoyl that is unsubstituted or mono- or poly-substituted by halogen, methoxy, amino, hydroxy and/or by cyano; benzoyl that is unsubstituted or substituted by one or more substituents from the group halogen, C₁-C₄alkyl, methoxy, amino, hydroxy and cyano; carboxy, (methoxy, ethoxy or butoxy)-carbonyl, carbamoyl, N-isopropylcarbamoyl, N-phenylcarbamoyl, N-pyrrolidylcarbonyl, nitro or amino; and pharmaceutically acceptable salts thereof.
   Preferred compounds of this group are:
   (1) 4-(2,4-dichlorobenzyloxy)-3-[1-(1-imidazolyl)-butyl]-benzonitrile,
   (2) (4-(4-bromobenzyloxy)-3-[1-(1-imidazolyl)-butyl]-phenyl pentyl ketone,
   (3) 4-(4-bromobenzyloxy)-3-[1-(1-imidazolyl)-butyl]-benzanilide,
   (4) 4-(4-bromobenzyloxy)-3-[1-(1-imidazolyl)-butyl]-benzoic acid,
   (5) 3-(2,4-dichlorobenzyloxy)-4-[1-(1-imidazolyl)-butyl]-benzonitrile,
   (6) 3-(2,4-dichlorobenzyloxy)-4-[1-(1-imidazolyl)-butyl]-benzoic acid methyl ester,
   (7) 3-(2,4-dichlorobenzyloxy)-4-[1-(1-imidazolyl)-butyl]-benzoic acid,
   (8) 3-(3-bromobenzyloxy)-4-[1-(1-imidazolyl)-butyl]-benzonitrile,
   (9) 4-(3-bromobenzyloxy)-3-[1-(1-imidazolyl)-butyl]-benzonitrile,
   (10) 3-(4-bromobenzyloxy)-4-[1-(1-imidazolyl)-butyl]-benzoic acid,
   (11) 3-(4-bromobenzyloxy)-4-[1-(1-imidazolyl)-butyl]-benzanilide,
   (12) 3-(4-bromobenzyloxy)-4-[1-(1-imidazolyl)-butyl]-phenyl pentyl ketone,
   (13) 4-(4-bromobenzyloxy)-3-[1-(1-imidazolyl)-butyl]-benzonitrile,
   (14) 3-(4-bromobenzyloxy)-4-[1-(1-imidazolyl)-butyl]-benzonitrile,
   (15) 4-nitro-2-[1-(1-imidazolyl)-butyl]-phenyl-(2,4-dichlorobenzyl) ether,
   (16) 4-amino-2-[1-(1-imidazolyl)-butyl]-phenyl-(2,4-dichlorobenzyl) ether,
   (17) (2,4-dichlorobenzyl)-[2-(1-imidazolyl-methyl)-4-nitrophenyl] ether.
(m) The compounds of formula I as defined in EP-A-337 928. These are especially the compounds of formula I 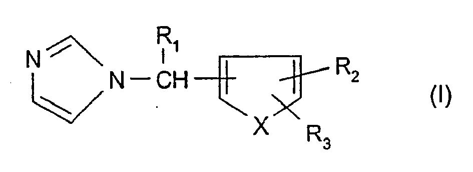 wherein R₁ is hydrogen, methyl, ethyl, propyl, propenyl, isopropyl, butyl, hexyl, octyl, decyl, cyclopentyl, cyclohexyl, cyclopentylmethyl, cyclohexylmethyl or benzyl, R₂ is hydrogen, halogen, cyano, methyl, hydroxymethyl, cyanomethyl, methoxymethyl, pyrrolidinylmethyl, carboxy, (methoxy, ethoxy or butoxy)-carbonyl, carbamoyl, N-isopropylcarbamoyl, N-phenylcarbamoyl, N-pyrrolidylcarbonyl; C₂-C₁₀alkanoyl that is unsubstituted or mono- or poly-substituted by halogen, methoxy, ethoxy, amino, hydroxy and/or by cyano; or benzoyl that is unsubstituted or substituted by one or more substituents from the group halogen, C₁-C₄alkyl, methoxy, ethoxy, amino, hydroxy and cyano, R₃ is hydrogen, benzyloxy, 3-bromo-, 4-bromo-, 4-chloro-, 2,3-, 2,4-, 4,5- or 4,6-dichloro-benzyloxy, and X is -CH=N-; -CH=N(-O)- or -S-; and pharmaceutically acceptable salts thereof.
   Preferred compounds of this group are:
   (1) 5-[1-(1-imidazolyl)-butyl]-thiophene-2-carbonitrile,
   (2) 2-[1-(1-imidazolyl)-butyl]-thiophene-4-carbonitrile,
   (3) 2-[1-(1-imidazolyl)-butyl]-4-bromo-thiophene,
   (4) 2-[1-(1 -imidazolyl)-butyl]-5-bromo-thiophene,
   (5) 5-[1-(1-imidazolyl)-butyl]-2-thienyl pentyl ketone,
   (6) 5-[1-(1-imidazolyl)-butyl]-2-thienyl ethyl ketone,
   (7) 5-(4-chlorobenzyloxy)-4-[1-(1-imidazolyl)-pentyl]-pyridine-2-carbonitrile,
   (8) 3-(4-chlorobenzyloxy)-4-[1-(1-imidazolyl)-pentyl]-pyridine-2-carbonitrile,
   (9) 3-(4-chlorobenzyloxy)-4-[1-(1-imidazolyl)-pentyl]-pyridine-N-oxide,
   (10) 3-(4-chlorobenzyloxy)-4-[1-(1-imidazolyl)-pentyl]-pyridine.
(n) The compounds of formula I as defined in EP-A-340 153. These are especially the compounds of formula I 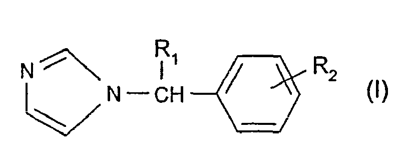 wherein R₁ is hydrogen, methyl, ethyl, propyl, propenyl, isopropyl, butyl, hexyl, octyl, decyl, cyclopentyl, cyclohexyl, cyclopentylmethyl, cyclohexylmethyl or benzyl, and R₂ is a radical from the group methyl, ethyl, propyl, benzyl, phenyl and ethenyl that is substituted by hydroxy, cyano, methoxy, butoxy, phenoxy, amino, pyrrolidinyl, carboxy, lower alkoxycarbonyl or by carbamoyl; or R₂ is formyl or derivatised formyl that can be obtained by reaction of the formyl group with an amine or amine derivative from the group hydroxylamine, O-methylhydroxylamine, O-ethylhydroxylamine, O-allylhydroxylamine, O-benzylhydroxylamine, O-4-nitrobenzyloxyhydroxylamine, O-2,3,4,5,6-pentafluorobenzyloxyhydroxylamine, semicarbazide, thiosemicarbazide, ethylamine and aniline; acetyl, propionyl, butyryl, valeryl, caproyl; benzoyl that is unsubstituted or substituted by one or more substituents from the group halogen, C₁-C₄alkyl, methoxy, amino, hydroxy and cyano; carboxy, (methoxy, ethoxy or butoxy)-carbonyl, carbamoyl, N-isopropylcarbamoyl, N-phenylcarbamoyl or N-pyrrolidylcarbonyl; and pharmaceutically acceptable salts thereof.
   Preferred compounds of this group are:
   (1) 4-(1-(1-imidazolyl)-butyl)-benzoic acid methyl ester,
   (2) 4-(1-(1-imidazolyl)-butyl)-benzoic acid butyl ester,
   (3) 4-(1-(1-imidazolyl)-butyl)-phenyl-acetonitrile,
   (4) 4-(1-(1-imidazolyl)-butyl)-benzaldehyde,
   (5) 4-(1-(1-imidazolyl)-butyl)-benzyl alcohol,
   (6) {4-[1-(1-imidazolyl)-butyl]-phenyl}-2-propyl ketone,
   (7) 4-[1-(1-imidazolyl)-butyl]-phenyl propyl ketone,
   (8) 4-[1-(1-imidazolyl)-butyl]-phenyl butyl ketone,
   (9) 4-[1-(1-imidazolyl)-butyl]-phenyl pentyl ketone,
   (10) 4-[1-(1-imidazolyl)-butyl]-phenyl hexyl ketone.
(o) The compounds of formula I as defined in DE-A-4 014 006. These are especially the compounds of formula I 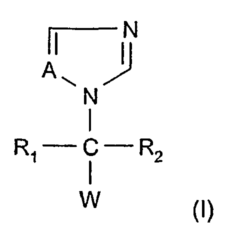 wherein A is an N-atom or a CH radical and W is a radical of the formula 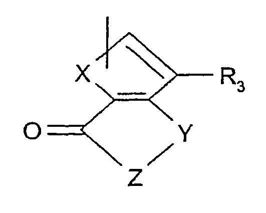 wherein X is an oxygen or a sulfur atom or a -CH=CH- group and Y is a methylene group, an oxygen or a sulfur atom and Z is a -(CH₂)ₙ- group wherein n = 1, 2 or 3 and either
   a) R₃ in W is a hydrogen atom and R₁ and R₂, independently of one another, are each a hydrogen atom, a C₁- to C₁₀alkyl group or a C₃- to C₇cycloalkyl group, or
   b) R₂ is as defined under a) and R₁ together with R₃ forms a -(CH₂)ₘ- group wherein m = 2, 3 or 4,
   and their pharmaceutically acceptable addition salts with acids.
   Preferred compounds of this group are:
   (1) 5-[1-(1-imidazolyl)-butyl]-1-indanone,
   (2) 7-[1-(1-imidazolyl)-butyl]-1-indanone,
   (3) 6-[1-(1-imidazolyl)-butyl]-1-indanone,
   (4) 6-(1-imidazolyl)-6,7,8,9-tetrahydro-1H-benz[e]inden-3(2H)-one,
   (5) 2-[1-(1-imidazolyl)-butyl]-4,5-dihydro-6-oxo-cyclopenta[b]-thiophene,
   (6) 6-[1-(1-imidazolyl)-butyl]-3,4-dihydro-2H-naphthalen-1-one,
   (7) 2-[1-(1-imidazolyl)-butyl]-6,7-dihydro-5H-benzo[b]thiophen-4-one,
   (8) 6-[1-(1-imidazolyl)-butyl]-2H-benzo[b]furan-3-one,
   (9) 5-[cyclohexyl-(1-imidazolyl)-methyl]-1-indanone,
   (10) 2-[1-(1-imidazolyl)-butyl]-4,5-dihydro-6H-benzo[b]thiophen-7-one,
   (11) 5-[1-(1-imidazolyl)-1-propyl-butyl]-1-indanone,
   (12) 2-[1-(1-imidazolyl)-butyl]-4,5-dihydro-6H-benzo[b]thiophen-7-one,
   (13) 2-[1-(1-imidazolyl)-butyl]-4,5-dihydro-6-oxo-cyclopenta[b]-thiophene,
   (14) 5-(1-imidazolylmethyl)-1-indanone,
   (15) 5-[1-(1,2,4-triazolyl)-methyl]-1-indanone.
(p) The compounds of formula I as disclosed in DE-A-3 926 365. These are especially the compounds of formula I 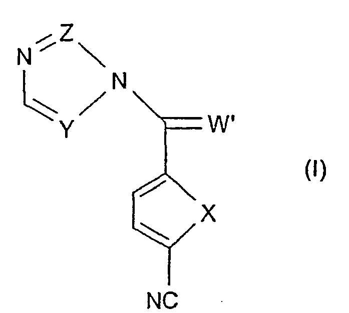 wherein W' is a cyclopentylidene, cyclohexylidene, cycloheptylidene or 2-adamantylidene radical, X is the grouping -CH=CH-, an oxygen or a sulfur atom, and Y and Z, independently of one another, are each a methine group (CH) or a nitrogen atom, and their pharmaceutically acceptable addition salts with acids.
   Preferred compounds of this group are:
   (1) 4-[1-cyclohexylidene-1-(imidazolyl)-methyl]-benzonitrile,
   (2) 4-[1-cyclopentylidene-1-(imidazolyl)-methyl]-benzonitrile,
   (3) 4-[1-cycloheptylidene-1-(imidazolyl)-methyl]-benzonitrile,
   (4) 4-[2-adamantylidene-1-(imidazolyl)-methyl]-benzonitrile,
   (5) 4-[1-cyclohexylidene-1-(1,2,4-triazolyl)-methyl]-benzonitrile,
   (6) 4-[1-cyclopentylidene-1-(1,2,4-triazolyl)-methyl]-benzonitrile,
   (7) 4-[1-cycloheptylidene-1-(1,2,4-triazolyl)-methyl]-benzonitrile,
   (8) 4-[2-adamantylidene-1-(1,2,4-triazolyl)-methyl]-benzonitrile,
   (9) 4-[1-cyclohexylidene-1-(1,2,3-triazolyl)-methyl]-benzonitrile,
   (10) 4-[1-cyclopentylidene-1-(1,2,3-triazolyl)-methyl]-benzonitrile,
   (11) 5-[cyclohexylidene-1-imidazolylmethyl]-thiophene-2-carbonitrile.
(q) The compounds of formula I as defined in DE-A-3 740 125. These are especially the compounds of formula I 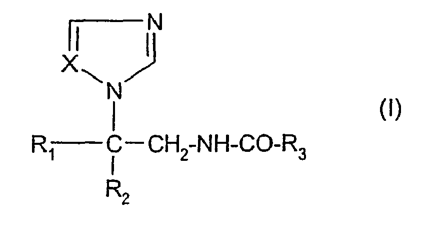 wherein X is CH or N, R₁ and R₂ are identical or different and are each phenyl or halophenyl, and R₃ is C₁-C₄alkyl; C₁-C₄alkyl substituted by CN, C₁-C₄alkoxy, benzyloxy or by C₁-C₄alkoxy-(mono-, di- or tri-)ethyleneoxy; C₁-C₄alkoxy, phenyl; phenyl that is substituted by halogen or by cyano; a C₅-C₇cycloalkyl group that is optionally condensed by benzene, or is thienyl, pyridyl or 2- or 3-indolyl; and pharmaceutically acceptable acid addition salts thereof.
   A preferred compound of this group is:
   (1) 2,2-bis(4-chlorophenyl)-2-(1H-imidazol-1-yl)-1-(4-chlorobenzoyl-amino)ethane.
(r) The compounds of formula I as defined in EP-A-293978. These are especially the compounds of formula I 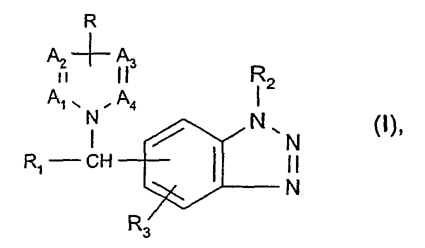 pharmaceutically acceptable salts and stereochemically isomeric forms thereof, wherein -A₁=A₂-A₃=A₄- is a divalent radical selected from -CH=N-CH=CH-, -CH=N-CH=N- and -CH=N-N=CH-, R is hydrogen or C₁-C₆alkyl; R₁ is hydrogen, C₁-C₁₀alkyl, C₃-C₇cycloalkyl, Ar₁, Ar₂-C₁-C₆alkyl, C₂-C₆alkenyl or C₂-C₆alkynyl; R₂ is hydrogen; C₁-C₁₀alkyl that is unsubstituted or substituted by Ar₁; C₃-C₇cycloalkyl, hydroxy, C₁-C₆alkoxy, Ar₁, C₂-C₆alkenyl, C₂-C₆alkynyl, C₃-C₇cycloalkyl, bicyclo[2.2.1]heptan-2-yl, 2,3-dihydro-1H-indenyl, 1,2,3,4-tetrahydronaphthyl, hydroxy; C₂-C₆alkenyloxy that is unsubstituted or substituted by Ar₂; C₂₋C₆alkynyloxy; pyrimidyloxy; di(Ar₂)methoxy, (1-C₁-C₄alkyl-4-piperidinyl)oxy, C₁-C₁₀alkoxy; or C₁-C₁₀alkoxy that is substituted by halogen, hydroxy, C₁-C₆alkyloxy, amino, mono- or di-(C₁₋C₆alkyl)amino, trifluoromethyl, carboxy, C₁-C₆alkoxycarbonyl, Ar₁, Ar₂-O-, Ar₂-S-, C₃₋C₇cycloalkyl, 2,3-dihydro-1,4-benzodioxinyl, 1 H-benzimidazolyl, C₁-C₄alkyl-substituted 1 H-benzimidazolyl, (1,1'-biphenyl)-4-yl or by 2,3-dihydro-2-oxo-1H-benzimidazolyl; and R₃ is hydrogen, nitro, amino, mono- or di-(C₁-C₆alkyl)amino, halogen, C₁-C₆alkyl, hydroxy or C₁₋C₆alkoxy; wherein Ar₁ is phenyl, substituted phenyl, naphthyl, pyridyl, aminopyridyl, imidazolyl, triazolyl, thienyl, halothienyl, furanyl, C₁-C₆a]kylfuranyl, halofuranyl or thiazolyl;
   wherein Ar₂ is phenyl, substituted phenyl or pyridyl; and wherein "substituted phenyl" is phenyl that is substituted by up to 3 substituents in each case selected independently of one another from the group consisting of halogen, hydroxy, hydroxymethyl, trifluoromethyl, C₁₋C₆alkyl, C₁-C₆alkoxy, C₁-C₆alkoxycarbonyl, carboxy, formyl, hydroxyiminomethyl, cyano, amino, mono- and di-(C₁-C₆alkyl)amino and nitro.
   Preferred compounds of this group are:
   (1) 6-[(1 H-imidazol-1-yl)-phenylmethyl]-1-methyl-1 H-benzotriazole,
   (2) 6-[(4-chlorophenyl)(1H-1,2,4-triazol-1-yl)methyl]-1-methyl-1 H-benzotriazole.
(s) The compounds of formula II as defined in EP-A-250198, especially
   (1) 2-(4-chlorophenyl)-1,1-di(1,2,4-triazol-1-ylmethyl)ethanol,
   (2) 2-(4-fluorophenyl)-1,1-di(1,2,4-triazol-1-ylmethyl)ethanol,
   (3) 2-(2-fluoro-4-trifluoromethylphenyl)-1,1-di(1,2,4-triazol-1-ylmethyl)ethanol,
   (4) 2-(2,4-dichlorophenyl)-1,1-di(1,2,4-triazol-1-ylmethyl)ethanol,
   (5) 2-(4-chlorophenyl)-1,1-di(1,2,4-triazol-1-ylmethyl)-ethanol,
   (6) 2-(4-fluorophenyl)-1,1-di(1,2,4-triazol-1-yl-methyl)ethanol.
(t) The compounds of formula I as defined in EP-A-281283, especially
   (1) (1R*,2R*)-6-fluoro-2-(4-fluorophenyl)-1,2,3,4-tetrahydro-1-(1H-1,2,4-triazol-1-ylmethyl)-naphthalene,
   (2) (1R*,2R*)-6-fluoro-2-(4-fluorophenyl)-1,2,3,4-tetrahydro-1-(1 H-imidazolylmethyl)naphthalene,
   (3) (1R*,2R*)- and (1R*,2S*)-2-(4-fluorophenyl)-1,2,3,4-tetrahydro-1-(1H-1,2,4-triazol-1-ylmethyl)naphthalene-6-carbonitrile,
   (4) (1R*,2R*) and (1R*,2S*)-2-(4-fluorophenyl)-1,2,3,4-tetrahydro-1-(1H-imidazolylmethyl)naphthalene-6-carbonitrile,
   (5) (1R*,2R*)- and (1R*,2S*)-1,2,3,4-tetrahydro-1-(1H-1,2,4-triazol-1-ylmethyl)naphthalene-2,6-dicarbonitrile,
   (6) (1R*,2R*)- and (1R*,2S*)-1,2,3,4-tetrahydro-1-(1H-imidazol-1-ylmethyl)naphthalene-2,6-dicarbonitrile,
   (7) (1R*,2S*)-2-(4-fluorophenyl)-1,2,3,4-tetrahydro-1-(5-methyl-1H-imidazolylmethyl)-naphthalene-6-carbonitrile.
(u) The compounds of formula I as defined in EP-A-296749, especially
   (1) 2,2'-[5-(1H-1,2,4-triazol-1-ylmethyl)-1,3-phenylene]di(2-methylpropiononitrile),
   (2) 2,2'-[5-(imidazol-1-ylmethyl)-1,3-phenylene]di(2-methylpropiononitrile),
   (3) 2-[3-(1-hydroxy-1-methylethyl)-5-(5H-1,2,4-triazol-1-ylmethyl)phenyl]-2-methylpropiononitrile,
   (4) 2,2'-[5-dideuterio(1H-1,2,4-triazol-1-yl)methyl-1,3-phenylene]di(2-trideuteriomethyl-3,3,3-trideuteriopropiononitrile),
   (5) 2,2'-[5-dideuterio(1H-1,2,4-triazol-1-yl)methyl-1,3-phenylene]di(2-methylpropiononitrile).
(v) The compounds of formula I as defined in EP-A-299683, especially
   (1) (Z)-α-(1,2,4-triazo)-1-ylmethyl)stilbene-4,4'-dicarbonitrile,
   (2) (Z)-4'-chloro-α-(1,2,4-triazol-1-ylmethyl)stilbene-4-carbonitrile,
   (3) (Z)-α-(1,2,4-triazol-1-ylmethyl)-4'-(trifluoromethyl)stilbene-4-carbonitrile,
   (4) (E)-β-fluoro-α-(1,2,4-triazol-1-ylmethyl)stilbene-4,4'-dicarbonitrile,
   (5) (Z)-4'-fluoro-α-(imidazol-1-ylmethyl)stilbene-4-carbonitrile,
   (6) (Z)-2',4'-dichloro-α-(imidazol-1-ylmethyl)stilbene-4-carbonitrile,
   (7) (Z)-4'-chloro-α-(imidazol-1-ylmethyl)stilbene-4-carbonitrile,
   (8) (Z)-a-(imidazol-1-ylmethyl)stilbene-4,4'-dicarbonitrile,
   (9) (Z)-α-(5-methyfimidazol-1-ylmethyl)stilbene-4,4'-dicarbonitrile,
   (10) (Z)-2-[2-(4-cyanophenyl)-3-(1,2,4-triazol-1-yl)propenyl]pyridine-5-carbonitrile.
(w) The compounds of formula I as defined in EP-A-299684, especially
   (1) 2-(4-chlorobenzyl)-2-fluoro-1,3-di(1,2,4-triazol-1-yl)propane,
   (2) 2-fluoro-2-(2-fluoro-4-chlorobenzyl)-1,3-di(1,2,4-triazol-1-yl)propane,
   (3) 2-fluoro-2-(2-fluoro-4-trifluoromethylbenzyl)-1,3-di(1,2,4-triazol-1-yl)propane,
   (4) 3-(4-chlorophenyl)-1-(1,2,4-triazol-1-yl)-2-(1,2,4-triazol-1-ylmethyl)butan-2-ol,
   (5) 2-(4-chloro-α-fluorobenzyl)-1,3-di(1,2,4-triazol-1-yl)propan-2-ol,
   (6) 2-(4-chlorobenzyl)-1,3-bis(1,2,4-triazol-1-yl)propane,
   (7) 4-[2-(4-chlorophenyl)-1,3-di(1,2,4-triazol-1-ylmethyl)ethoxymethyl]-benzonitrile,
   (8) 1-(4-fluorobenzyl)-2-(2-fluoro-4-trifluoromethylphenyl)-1,3-di(1,2,4-triazo)-1-yl)propan-2-ol,
   (9) 2-(4-chlorophenyl)-1-(4-fluorophenoxy)-1,3-di(1,2,4-triazol-1-yl)propan-2-ol,
   (10) 1-(4-cyanobenzyl)-2-(2,4-difluorophenyl)-1,3-di(1,2,4-triazol-1-yl)propan-2-ol,
   (11) 2-(4-chlorophenyl)-1-phenyl-1,3-di(1,2,4-triazol-1-yl)propan-2-ol.
(x) The compounds as defined in claim 1 of EP-A-316097, especially
   (1) 1,1-dimethyl-8-(1H-1,2,4-triazol-1-ylmethyl)-2(1H)-naphtho[2,1-b]furanone,
   (2) 1,2-dihydro-1,1-dimethyl-2-oxo-8-(1H-1,2,4-triazol-1-ylmethyl)naphtho[2,1-b]furan-7-carbonitrile,
   (3) 1,2-dihydro-1,1-dimethyl-2-oxo-8-(1H-1,2,4-triazol-1-ylmethyl)naphtho[2,1-b]furan-7-carboxamide,
   (4) 1,2-dihydro-1,1-dimethyl-2-oxo-8-[di(1H-1,2,4-triazol-1-yl)methyl]naphtho[2,1-b]furan-7-carbonitrile.
(y) The compounds of formula I as defined in EP-A-354689, especially
   (1) 4-[2-(4-cyanophenyl)-3-(1,2,4-triazol-1-yl)propyl]benzonitrile,
   (2) 4-[1-(4-chlorobenzyl)-2-(1,2,4-triazol-1-yl)ethyl]benzonitrile,
   (3) 4-[2-(1,2,4-triazol-1-yl)-1-(4-[trifluoromethyl]benzyl)ethyl]benzonitrile,
   (4) 4-[2-(1,2,4-triazol-1-yl)-1-(4-[trifluoromethoxy]benzyl)ethyl]benzonitrile.
(z) The compounds of formula (1) as defined in EP-A-354683, especially
   (1) 6-[2-(4-cyanophenyl)-3-(1,2,4-triazol-1-yl)-propyl]nicotinonitrile,
   (2) 4-[1-(1,2,4-triazol-1-yl-methyl)-2-(5-[trifluoromethyl]pyrid-2-yl)ethyl]benzonitrile.
   Examples of steroidal aromatase inhibitors that may be mentioned are:
   (aa) The compounds of formula I as defined in EP-A-181287. These are especially the compounds of formula I 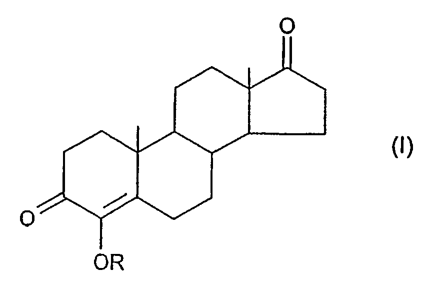 wherein R is hydrogen, acetyl, heptanoyl or benzoyl.
      A preferred compound of this group is:
      (1) 4-hydroxy-4-androstene-3,17-dione.
   (ab) The compounds as defined in the claims of US Patent 4322416, especially 10-(2-propynyl)-oestr-4-ene-3,17-dione.
   (ac) The compounds as defined in the claims of DE-A-3622841, especially 6-methylene-androsta-1,4-diene-3,17-dione.
   (ad) The compounds as defined in the claims of GB-A-2171100, especially 4-amino-androsta-1,4,6-triene-3,17-dione.
Also: (ae) androsta-1,4,6-triene-3,17-dione.
The general terms used to define the aromatase inhibitors mentioned above under (a) to (r) have the following meanings:
Organic radicals designated by the term "lower" contain up to and including 7, preferably up to and including 4, carbon atoms.
Acyl is especially lower alkanoyl.
Aryl is, for example, phenyl or 1- or 2-naphthyl, each of which is unsubstituted or substituted by lower alkyl, hydroxy, lower alkoxy, lower alkanoyloxy, amino, lower alkylamino, di-lower alkylamino, lower alkanoylamino or by halogen.
Any reference hereinbefore and hereinafter to a free bisphosphonate or a free aromatase inhibitor is to be understood as referring also to the corresponding pharmaceutically acceptable salts thereof, as appropriate and expedient.

The aromatase inhibitors can also be used in the form of their hydrates or include other solvents used for their crystallisation.

The most preferred aromatase inhibitor for use in the invention is 4-[α-(4-cyanophenyl)-1-(1,2,4-triazolyl)methyl]-benzonitrile (letrozole) or a pharmaceutically acceptable salt thereof.

Letrozole can be prepared as described in US 5,473,078. It can be administered, e.g., as described in US 4,978,672 or US 5,473,078, or in the form as it is marketed, e.g. under the trademark FEMARA^{™} or FEMAR^{™}.

Pharmaceutically acceptable salts of bisphosphonates and aromatase inhibitors are preferably salts with bases, conveniently metal salts derived from groups Ia, Ib, IIa and IIb of the Periodic Table of the Elements, including alkali metal salts, e.g. potassium and especially sodium salts, or alkaline earth metal salts, preferably calcium or magnesium salts, and also ammonium salts with ammonia or organic amines.

Especially preferred pharmaceutically acceptable salts of the N-bisphosphonates are those where one, two, three or four, in particular one or two, of the acidic hydrogens of the bisphosphonic acid are replaced by a pharmaceutically acceptable cation, in particular sodium, potassium or ammonium, in first instance sodium.

A very preferred group of pharmaceutically acceptable salts of the N-bisphosphonates is characterized by having one acidic hydrogen and one pharmaceutically acceptable cation, especially sodium, in each of the phosphonic acid groups.

The Agents of the Invention, i.e. the aromatase inhibitor and the bisphosphonate, are preferably used in the form of pharmaceutical preparations that contain the relevant therapeutically effective amount of each active ingredient (either separately or in combination) optionally together with or in admixture with inorganic or organic, solid or liquid, pharmaceutically acceptable carriers which are suitable for administration. The Agents of the Invention may be present in the same pharmaceutical compositions, though are preferably in separate pharmaceutical compositions. Thus the active ingredients may be administered at the same time (e.g. simultaneously) or at different times (e.g. sequentially) and over different periods of time, which may be separate from one another or overlapping.

The pharmaceutical compositions may be, for example, compositions for enteral, such as oral, rectal, aerosol inhalation or nasal administration, compositions for parenteral, such as intravenous or subcutaneous administration, or compositions for transdermal administration (e.g. passive or iontophoretic).

The particular mode of administration and the dosage may be selected by the attending physician taking into account the particulars of the patient, especially age, weight, life style, activity level, and disease state as appropriate as well as the particular aromatase inhibitor and bisphosphonate chosen.

The bisphosphonate pharmaceutical compositions may be adapted to oral or parenteral (especially intravenous, intra-arterial or transdermal) administration. Intravenous and oral, first and foremost intravenous, administration is considered to be of particular importance. Preferably the bisphosphonate active ingredient is in a parenteral form, most preferably an intravenous form.

The dosage of the bisphosphonate for use in the invention may depend on various factors, such as effectiveness and duration of action of the active ingredient, mode of administration, sex, age, weight and individual condition of the patient.

Normally the dosage is such that a single dose of the bisphosphonate active ingredient from 0.002 - 20.0 mg/kg, especially 0.01-10.0 mg/kg, is administered to a warm-blooded animal weighing approximately 75 kg. If desired, this dose may also be taken in several, optionally equal, partial doses.

"mg/kg" means mg drug per kg body weight of the mammal - including man - to be treated.

The dose mentioned above - either administered as a single dose (which is preferred) or in several partial doses - may be repeated, for example once daily, once weekly, once every month, or less frequently, e.g. once every three months, once every half year, once every year or more or at any interval there between. The dosing frequency for the bisphosphonate need not be an exact interval of, e.g. 1 month, 3 months, 6 months or 1 year, but may approximate to this interval; for instance, to allow for scheduling of doctors appointments, e.g. by +/- up to 14 days.

When the bisphosphonate is zoledronic acid, the preferred dosage is 4 or 5 mg once about every three (3) - six (6) months, preferably every six (6) months, beginning for example on the date of the first administration of aromatase inhibitor. However, the bisphosphonate may also be administered prior to or after the administration of the aromatase inhibitor. The zoledronic acid is generally administered intravenously over a 15-minute period.

Preferably, the bisphosphonates are administered in doses which are in the same order of magnitude as those used in the treatment of the diseases classically treated with bisphosphonic acid derivatives, such as Paget's disease, tumor-induced hypercalcemia or osteoporosis. In other words, preferably the bisphosphonic acid derivatives are administered in doses which would likewise be therapeutically effective in the treatment of Paget's disease, tumor-induced hypercalcemia or osteoporosis, i.e. preferably they are administered in doses which effectively inhibit bone resorption. For example, for the preferred nitrogen-containing bisphosphonates, e.g. zoledronic acid and salts thereof, doses of bisphosphonate in the range from about 0.5 to about 20 mg, preferably from about 1 to about 10 mg, may be used for treatment of human patients.

Bisphosphonate formulations in single dose unit form contain preferably from about 1% to about 90%, and formulations not in single dose unit form contain preferably from about 0.1% to about 20%, of the active ingredient. Single dose unit forms such as capsules, tablets or dragées contain e.g. from about 1 mg to about 500 mg of the active ingredient.

Preferably, the aromatase pharmaceutical compositions are adapted for oral or parenteral (especially oral) administration. Intravenous and oral, first and foremost oral, administration is considered to be of particular importance. Preferably the aromatase inhibitor active ingredient is in oral form.

Similarly the dosage of aromatase inhibitor administered is dependent on the species of warm-blooded animal (mammal), the body weight, age and individual condition, and on the form of administration. The applied dosage of the aromatase inhibitor may range between about 0.001 and 30.0 mg/kg, preferably between about 0.001 to 5 mg/kg.

Aromatase inhibitor formulations in single dose unit form contain preferably from about 1% to about 90%, and formulations not in single dose unit form contain preferably from about 0.1% to about 20%, of the active ingredient. Single dose unit forms such as capsules, tablets or dragées contain e.g. from about 1 mg to about 100 mg of the aromatase inhibitor.

Letrozole is preferably administered daily according to the package insert at a dose of 2.5 mg.

Pharmaceutical preparations comprising Agents of the Invention for enteral and parenteral administration are, for example, those in dosage unit forms, such as dragées, tablets or capsules and also ampoules. They are prepared in a manner known *per se,* for example by means of conventional mixing, granulating, confectioning, dissolving or lyophilizing processes. For example, pharmaceutical preparations for oral administration can be obtained by combining the active ingredient with solid carriers, where appropriate granulating a resulting mixture, and processing the mixture or granulate, if desired or necessary after the addition of suitable adjuncts, into tablets or dragée cores.

Other orally administrable pharmaceutical preparations are dry-filled capsules made of gelatin, and also soft, sealed capsules made of gelatin and a plasticiser, such as glycerol or sorbitol. The dry-filled capsules may contain the active ingredient in the form of a granulate, for example in admixture with fillers, such as lactose, binders, such as starches, and/or glidants, such as talc or magnesium stearate, and, where appropriate, stabilisers. In soft capsules the active ingredient is preferably dissolved or suspended in suitable liquids, such as fatty oils, paraffin oil or liquid polyethylene glycols, it being possible also for stabilisers to be added.

Parenteral formulations are especially injectable fluids that are effective in various manners, such as intravenously, intramuscularly, intraperitoneally, intranasally, intradermally or subcutaneously. Such fluids are preferably isotonic aqueous solutions or suspensions which can be prepared before use, for example from lyophilised preparations which contain the active ingredient alone or together with a pharmaceutically acceptable carrier. The pharmaceutical preparations may be sterilised and/or contain adjuncts, for example preservatives, stabilisers, wetting agents and/or emulsifiers, solubilisers, salts for regulating the osmotic pressure and/or buffers.

Suitable formulations for transdermal application include an effective amount of the active ingredient with carrier. Advantageous carriers include absorbable pharmaceutically acceptable solvents to assist passage through the skin of the host. Characteristically, transdermal devices are in the form of a bandage comprising a backing member, a reservoir containing the compound optionally with carriers, optionally a rate controlling barrier to deliver the active ingredient of the skin of the host at a controlled and predetermined rate over a prolonged period of time, and means to secure the device to the skin.

When the combination partners of the present invention are applied in the form as marketed as single drugs, their dosage and mode of administration can take place in accordance with the information provided on the package insert of the respective marketed drug in order to result in the beneficial effect described herein, if not mentioned herein otherwise.

The following examples are intended to illustrate the invention and are not to be construed as being limitations thereon.

### EXAMPLES

### A. Formulation Examples

### Example 1:

Capsules containing coated pellets of active ingredient, for example, disodium pamidronate pentahydrate, as active ingredient:

| | |
|---|---|
| Core pellet: | |
| active ingredient (ground) | 197.3 mg |
| Microcrystalline cellulose | 52.7 mg |
| (Avicel^{®} PH 105) | 250.0 mg |

| | |
|---|---|
| + Inner coating: | |
| Cellulose HP-M 603 | 10.0 mg |
| Polyethylene glycol | 2.0 mg |
| Talc | 8.0 mg |
| | 270.0 mg |

| | |
|---|---|
| + Gastric juice-resistant outer coating: | |
| Eudragit^{®} L 30 D (solid) | 90.0 mg |
| Triethyl citrate | 21.0 mg |
| Antifoam^{®} AF | 2.0 mg |
| Water | |
| Talc | 7.0 mg |
| | 390.0 mg |

A mixture of disodium pamidronate with Avicel^{®} PH 105 is moistened with water and kneaded, extruded and formed into spheres. The dried pellets are then successively coated in the fluidized bed with an inner coating, consisting of cellulose HP-M 603, polyethylene glycol (PEG) 8000 and talc, and the aqueous gastric juice-resistant coat, consisting of Eudragit^{®} L 30 D, triethyl citrate and Antifoam^{®} AF. The coated pellets are powdered with talc and filled into capsules (capsule size 0) by means of a commercial capsule filling machine, for example Höfliger and Karg.

### Example 2:

Monolith adhesive transdermal system, containing as active ingredient, for example, 1-hydroxy-2-(imidazol-1-yl)-ethane-1,1 -diphosphonic acid:

### Composition:

| | |
|---|---|
| polyisobutylene (PIB) 300 | 5.0 g |
| (Oppanol B1, BASF) | |
| PIB 35000 | 3.0 g |
| (Oppanol B10, BASF) | |
| PIB 1200000 | 9.0 g |
| (Oppanol B100, BASF) | |
| hydrogenated hydrocarbon resin | 43.0 g |
| (Escorez 5320, Exxon) | |
| 1-dodecylazacycloheptan-2-one | 20.0 g |
| (Azone, Nelson Res., Irvine/CA) | |
| active ingredient | 20.0g |
| Total | 100.0 g |

### Preparation:

The above components are together dissolved in 150 g of special boiling point petroleum fraction 100-125 by rolling on a roller gear bed. The solution is applied to a polyester film (Hostaphan, Kalle) by means of a spreading device using a 300 mm doctor blade, giving a coating of about 75 g/m². After drying (15 minutes at 60°C), a silicone-treated polyester film (thickness 75 mm, Laufenberg) is applied as the peel-off film. The finished systems are punched out in sizes in the wanted form of from 5 to 30 cm² using a punching tool. The complete systems are sealed individually in sachets of aluminized paper.

### Example 3:

Vial containing 1.0 mg dry, lyophilized 1-hydroxy-2-(imidazol-1-yl)ethane-1,1-diphosphonic acid (mixed sodium salts thereof). After dilution with 1 ml of water, a solution (concentration 1 mg/ml) for i.v. infusion is obtained.

### Composition:

| | |
|---|---|
| active ingredient (free diphosphonic acid) | 1.0 mg |
| mannitol | 46.0 mg |
| Trisodium citrate x 2 H₂O | ca. 3.0 mg |
| water | 1 ml |
| water for injection | 1 ml. |

In 1 ml of water, the active ingredient is titrated with trisodium citrate x 2 H₂O to pH 6.0. Then, the mannitol is added and the solution is lyophilized and the lyophilisate filled into vial.

### Example 4:

Ampoule containing active ingredient, for instance disodium pamidronate pentahydrate dissolved in water. The solution (concentration 3 mg/ml) is for i.v. infusion after dilution.

### Composition:

| | |
|---|---|
| active ingredient | 19.73 mg |
| (△ 5.0 mg of anhydrous active ingredient) | |
| mannitol | 250 mg |
| water for injection | 5 ml. |

### Example 5:

Film coated tablets containing 2.5 mg of for example letrozole as active ingredient:

| **Component** | **Function** | **Amount/Tablet (mg)** |
|---|---|---|
| **Core** | | |
| Letrozole | Active ingredient | 2.50 |
| Colloidal Anhydrous Silica | Glidant | 0.5 |
| Microcrystalline Cellulose | Binder (dry) | 20.00 |
| Lactose Monohydrate, cryst. | Diluent | 61.50 |
| Magnesium Stearate | Lubricant | 1.00 |
| Maize Starch | Diluent, disintegrant | 9.50 |
| Sodium Starch Glycolate (Sodium Carboxymethyl Starch) | Disintegrant | 5.00 |
| | | |
| **Film-Coat** | | |
| Methylhydroxypropylcellulose | Film-forming agent | 1.838 |
| Iron oxide, yellow | Color pigment | 0.249 |
| Polyethylene Glycol 8000 | Plasticizer | 0.333 |
| Talc, PH | Anti-adhesive and opacifier | 1.331 |
| Titanium Dioxide, PH | Pigment and opacifier | 0.249 |
| Purified Water* | Coating solvent | qs |
| Ethanol with 5% Isopropanol* | Coating solvent | qs |

| | | |
|---|---|---|
| *Removed during processing | | |

### Preparation:

1. Letrozole is mixed with maize starch in a tumble mixer, screened, mixed and screened again.
2. The pre-mixture is blended in a tumble mixer with screened colloidal anhydrous silica, microcrystalline cellulose, lactose monohydrate and sodium starch glycolate, screened and blended again.
3. After admixing of magnesium stearate the homogeneous tablet mass is compressed into tablets containing the stated amount of active substance.
4. The tablets are coated with a lacquer composed of the excipients stated in the formula above, dissolved or suspended in purified water and small amounts of ethanol with 5% isopropyl alcohol.

### Example 6: Intravenous administration of zoledronic acid prevents the bone loss and reduction of mechanical properties induced by aromatase inhibition or surgical ovariectomy in rats

It was investigated whether bone loss induced in rats by either ovariectomy (OVX) or the aromatase inhibitor letrozole (Let) could be prevented by the bisphosphonate zoledronic acid (Zol).

### Material and Methods:

Adult, skeletally mature, 8-month-old female Wistar rats were assigned to the following treatment groups (n=10): Sham (vehicle), OVX, Let-treated, OVX + Zol and Let + Zol. Zol was injected into the tail vein as a single dose of 0.8, 4 or 20 µg/kg 3 days before OVX or before initiating daily oral dosing of Let (1 mg/kg) for 24 weeks. Changes in total and cancellous bone mineral density (BMD) and cortical thickness in the proximal tibia metaphysis were monitored non-invasively by peripheral quantitative computed tomography (pQCT) at 0, 2, 4, 8, 12, 16, 20 and 24 weeks. Both tibiae, femora and lumbar vertebrae L1-L6 were harvested at necropsy in order to carry out biomechanical testing.

### Results:

**Total BMD** decreased rapidly after OVX and reached a plateau at around 20 weeks (-16%) (*P*<0.01 at all time points vs sham). A single iv injection of 0.8, 4 or 20 µg/kg zoledronic acid delayed bone loss significantly for 8, 20 and 24 weeks, respectively, with the highest dose being fully protective over the entire 24-week duration of the study (NS vs sham).
**Mean cortical thickness** decreased rapidly in OVX rats (*P*<0.01 at all time points vs sham, -25% at 24 weeks). A marginal protective effect of a single iv dose of 0.8 µg/kg zoledronic acid was observed at 2 weeks (*P*<0.05 vs OVX). At 4 and 20 µg/kg, cortical thickness was significantly better than in OVX controls for 12 and 24 weeks, respectively. The highest dose was fully protective for the full duration of the study (NS vs sham at all time points). Cancellous bone loss in OVX rats progressed rapidly, reaching a plateau at 24 weeks (*P*<0.01 at all time points vs sham, -46% at 24 weeks). A single iv injection of 0.8 µg/kg zoledronic acid delayed cancellous bone loss marginally but significantly for 12 weeks. At doses of 4 and 20 µg/kg, cancellous bone mineral density was significantly better than in OVX controls for 24 weeks. The highest dose was fully protective for 20 weeks, but mild cancellous bone loss was visible at 24 weeks (NS vs sham).
**Total BMD** decreased slowly after administration of letrozole until week 4 and more rapidly thereafter to then slow down again at 12 weeks (-13% at 24 weeks) (*P*<0.01 at all time points vs sham). A single iv injection of 0.8 µg/kg zoledronic acid delayed bone loss marginally but significantly for 4 weeks. At 4 and 20 µg/kg, zoledronic acid delayed bone loss significantly for 24 weeks (*P*<0.01 vs letrozole for both doses) with the highest dose being fully protective over the entire 24-week duration of the study (NS vs sham).
**Mean cortical thickness** decreased rapidly in letrozole-treated rats (*P*<0.01 at all time points vs sham, -21 % at 24 weeks). A marginal but significant protective effect of a single iv dose of 0.8 µg/kg zoledronic acid was observed at 2 weeks (*P*<0.05 vs letrozole). At 4 µg/kg, zoledronic acid fully prevented cortical thinning for 4 weeks, but bone loss set in thereafter, with values staying significantly above the letrozole control until week 20 (*P*<0.01 vs letrozole at all time points). The highest zoledronic acid dose fully protected against cortical thinning for 24 weeks (*P*<0.01 vs letrozole and NS vs sham at all time points).
Cancellous bone loss in letrozole-treated rats progressed rapidly, reaching a plateau at 24 weeks (*P*<0.01 at all time points vs sham, -26% at 24 weeks). A single iv injection of 0.8 µg/kg zoledronic acid delayed cancellous bone loss marginally but significantly for 8 weeks (*P*<0.05 vs letrozole). At 4 µg/kg, zoledronic acid fully prevented bone loss for 8 weeks, but bone loss set in thereafter (*P*<0.01 vs sham at 16 to 24 weeks). However, cancellous bone mineral density remained significantly higher than in letrozole-treated rats for 24 weeks. The highest dose (20 µg/kg) was fully protective for 24 weeks (*P*<0.01 vs letrozole and NS vs sham at all time points).
**Mechanical properties** of the 5th lumbar vertebral body were evaluated in compression after removal of the epiphyseal ends, posterior pedicle arch and spinous process on a 4 mm segment with plano-parallel ends. Ultimate bone strength was significantly reduced after OVX (*P*<0.05) and decreased after letrozole administration (NS). Zoledronic acid dose-dependently improved the ultimate bone strength in both situations.

### Discussion:

Our data indicate for the first time that in rats, Zol dose-dependently protects against cancellous bone loss, cortical thinning and reduction of bone strength induced by daily oral Let. Zol, at a dose of 20 µg/kg, fully protects against Let-induced bone loss for at least 24 weeks. These data support the use ofbisphosphonates in general and Zol in particular in preventing or reducing bone loss in patients undergoing treatment with aromatase inhibitors in general and Let in particular.

## Claims

1. Use of (a) an aromatase inhibitor for the preparation of a medicament, for use in combination with (b) a N-bisphosphonate for treatment of a disease or condition which responds to aromatase inhibition, with the proviso that the disease or condition is not additionally treated with a chemotherapeutic agent.

2. The use according to claim 1 wherein the disease is a proliferative disease.

3. The use according claim 1 or 2 wherein the N-bisphosphonate is administered once every six months.

4. A combination which comprises (a) an aromatase inhibitor and (b) a N-bisphosphonate in which the active ingredients (a) and (b) are present in each case in free form or in the form of a pharmaceutically acceptable salt, for simultaneous, concurrent, separate or sequential use in the prevention of bone loss which is caused by the treatment with an aromatase inhibitor of a disease or condition which responds to aromatase inhibition.

5. Use of (a) an aromatase inhibitor for the preparation of a medicament, for use in combination with (b) a N-bisphosphonate for prevention of bone loss which is caused by the treatment with an aromatase inhibitor of a disease or condition which responds to aromatase inhibition.

6. The use according to anyone of claim 1 to 3 or 5 or the combination according to claim 4 wherein (a) the aromatase inhibitor is selected from exemestane, formestane, aminoglutethimide, vorozole, fadrozole, anastrozole, roglethimide, pyridoglutethimide, trilostane, testolactone, atamestane, 1-methyl-1,4-androstadiene-3,17-dione, ketokonazole and a compound of formula I 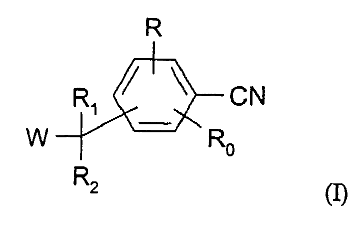 wherein R and R₀, independently of one another, are each hydrogen or lower alkyl, or R and R₀ at adjacent carbon atoms, together with the benzene ring to which they are bonded, form a naphthalene or tetrahydronaphthalene ring; wherein R₁ is hydrogen, lower alkyl, aryl, aryl-lower alkyl or lower alkenyl; R₂ is hydrogen, lower alkyl, aryl, aryl-lower alkyl, (lower alkyl, aryl or aryl-lower alkyl)-thio or lower alkenyl, or wherein R₁ and R₂ together are lower alkylidene or C₄-C₆alkylene; wherein W is 1-imidazolyl, 1-(1,2,4 or 1,3,4)-triazolyl, 3-pyridyl or one of the mentioned heterocyclic radicals substituted by lower alkyl; and aryl within the context of the above definitions has the following meanings: phenyl that is unsubstituted or substituted by one or two substituents from the group lower alkyl, lower alkoxy, hydroxy, lower alkanoyloxy, nitro, amino, halogen, trifluoromethyl, cyano, carboxy, lower alkoxycarbonyl, carbamoyl, N-lower alkylcarbamoyl, N,N-di-lower alkylcarbamoyl, lower alkanoyl, benzoyl, lower alkylsulfonyl, sulfamoyl, N-lower alkylsulfamoyl and N,N-di-lower alkylsulfamoyl; also thienyl, indolyl, pyridyl or furyl, or one of the four last-mentioned heterocyclic radicals monosubstituted by lower alkyl, lower alkoxy, cyano or by halogen; or a pharmaceutically acceptable salt thereof, and wherein "lower" means up to 7 carbon atoms.

7. The use or the combination according to claim 6 in which (a) the aromatase inhibitor is 4-[α-(4-cyanophenyl)-1-(1,2,4-triazolyl)methyl]-benzonitrile (letrozole) or a pharmaceutically acceptable salt thereof.

8. The use or the combination according to claim 7 wherein (b) the N-bisphosphonate is a compound of formula I 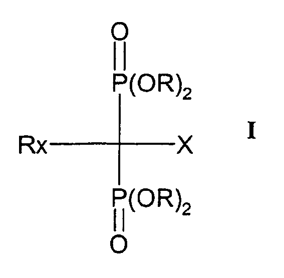 wherein
X is hydrogen, hydroxyl, amino, alkanoyl, or an amino group substituted by C₁-C₄ alkyl, or alkanoyl;
R is hydrogen or C₁-C₄ alkyl and
Rx is a side chain which contains an optionally substituted amino group, or a nitrogen containing heterocycle (including aromatic nitrogen-containing heterocycles),
or a pharmaceutically acceptable salt thereof or any hydrate thereof.

9. The use or combination according to claim 8 in which (b) the N-bisphosphonate is 2-(imidazol-1yl)-1-hydroxyethane-1,1-diphosphonic acid (zoledronic acid) or a pharmaceutically acceptable salt thereof.

10. The use according to anyone of claims 1 to 3 or 5 to 9 or the combination according to anyone of claims 4 to 9 wherein (a) the aromatase inhibitor is administered prior to the N-bisphosphonate.

11. The use according to claim 5 in a patient suffering from an estrogen dependent disorder.

12. The use according to claim 5 in a post menopausal woman with ER+ and/or PR+ breast cancer.

## Patentansprüche

1. Verwendung von (a) einem Aromatasehemmer zur Herstellung eines Arzneimittels zur Verwendung in Kombination mit (b) einem N-Bisphosphonat zur Behandlung einer Erkrankung oder eines Zustands, die/der auf eine Aromatasehemmung anspricht unter der Maßgabe, dass die Erkrankung oder der Zustand nicht zusätzlich mit einem chemotherapeutischen Mittel behandelt wird.

2. Verwendung nach Anspruch 1, wobei die Erkrankung für eine proliferative Erkrankung steht.

3. Verwendung nach Anspruch 1 oder 2, wobei das N-Bisphosphonat einmal alle sechs Monate verabreicht wird.

4. Kombination, die umfasst: (a) einen Aromatasehemmer und (b) ein N-Bisphosphonat, in der die wirksamen Inhaltsstoffe (a) und (b) in jedem Fall in freier Form oder in der Form eines pharmazeutisch annehmbaren Salzes vorliegen, zur gleichzeitigen, nebenläufigen, getrennten oder sequenziellen Verwendung bei der Prävention von Knochenverlust, der verursacht wird durch die Behandlung einer Erkrankung oder eines Zustands, die/der auf eine Aromatasehemmung anspricht, mit einem Aromatasehemmer.

5. Verwendung von (a) einem Aromatasehemmer zur Herstellung eines Arzneimittels, zur Verwendung in Kombination mit (b) einem N-Bisphosphonat zur Prävention von Knochenverlust, der verursacht wird durch die Behandlung einer Erkrankung oder eines Zustands, die/der auf eine Aromatasehemmung anspricht, mit einem Aromatasehemmer.

6. Verwendung nach einem der Ansprüche 1 bis 3 oder 5 oder Kombination nach Anspruch 4, wobei (a) der Aromatasehemmer ausgewählt ist aus Exemestan, Formestan, Aminoglutethimid, Vorozol, Fadrozol, Anastrozol, Roglethimid, Pyridoglutethimid, Trilostan, Testolacton, Atamestan, 1-Methyl-1,4-androstadien-3,17-dion, Ketokonazol und einer Verbindung der Formel I 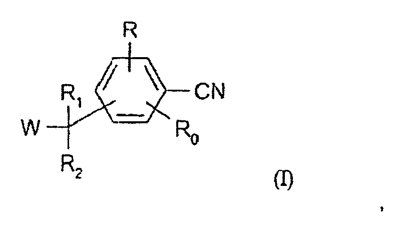 wobei R und R₀ unabhängig voneinander jeweils für Wasserstoff oder Niederalkyl stehen, oder R und R₀ an benachbarten Kohlenstoffatomen zusammen mit dem Benzolring, an den sie gebunden sind, einen Naphthalin- oder Tetrahydronaphtalinring bilden; wobei R₁ steht für Wasserstoff, Niederalkyl, Aryl, Arylniederalkyl oder Niederalkenyl; R₂ steht für Wasserstoff, Niederalkyl, Aryl, Arylniederalkyl, (Niederalkyl-, Aryl- oder Arylniederalkyl)-thio oder Niederalkenyl, oder wobei R₁ und R₂ zusammen für Niederalkyliden oder C₄-C₆-Alkylen stehen; wobei W steht für 1-Imidazolyl, 1-(1,2,4 oder 1,3,4)-Triazolyl, 3-Pyridyl oder einen der genannten heterocyclischen Reste, die durch Niederalkyl substituiert sind; und Aryl im Zusammenhang der obigen Definitionen die folgenden Bedeutungen aufweist: Phenyl, das unsubstituiert oder substituiert ist durch ein oder zwei Substituenten aus der Gruppe Niederalkyl, Niederalkoxy, Hydroxy, Niederalkanoyloxy, Nitro, Amino, Halogen, Trifluormethyl, Cyano, Carboxy, Niederalkoxycarbonyl, Carbamoyl, N-Niederalkylcarbamoyl, N,N-Diniederalkylcarbamoyl, Niederalkanoyl, Benzoyl, Niederalkylsulfonyl, Sulfamoyl, N-Niederalkylsulfamoyl und N,N-Diniederalkylsulfamoyl; auch Thienyl, Indolyl, Pyridyl oder Furyl, oder einer der vier zuletzt genannten heterocyclischen Reste, der monosubstituiert ist durch Niederalkyl, Niederalkoxy, Cyano oder durch Halogen; oder eines pharmazeutisch annehmbaren Salzes davon, und wobei "Nieder" bis zu 7 Kohlenstoffatome bedeutet.

7. Verwendung oder Kombination nach Anspruch 6, wobei (a) der Aromatasehemmer für 4-[α-(4-Cyanophenyl)-1-(1,2,4-triazolyl)methyl]-benzonitril (Letrozol) oder ein pharmazeutisch annehmbares Salz hiervon steht.

8. Verwendung oder Kombination nach Anspruch 7, wobei (b) das N-Bisphosphonat für eine Verbindung der Formel I steht 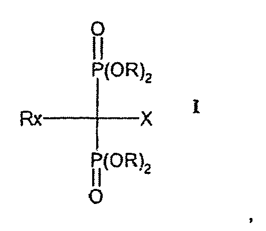 wobei
X steht für Wasserstoff, Hydroxyl, Amino, Alkanoyl oder eine Aminogruppe, die durch C₁-C₄-Alkyl substituiert ist, oder Alkanoyl;
R steht für Wasserstoff oder C₁-C₄-Alkyl und
Rₓ steht für eine Seitenkette, die eine gegebenenfalls substituierte Aminogruppe enthält, oder einen Stickstoff enthaltenden Heterocyclus (einschließlich aromatischer Stickstoff enthaltender Heterocyclen), oder ein pharmazeutisch annehmbares Salz davon oder ein beliebiges Hydrat davon.

9. Verwendung oder Kombination nach Anspruch 8, wobei (b) das N-Bisphosphonat für 2-(Imidazol-1yl)-1-hydroxyethan-1,1-diphosphonsäure (Zoledronsäure) oder ein pharmazeutisch annehmbares Salz davon steht.

10. Verwendung nach einem der Ansprüche 1 bis 3 oder 5 bis 9 oder Kombination nach einem der Ansprüche 4 bis 9, wobei (a) der Aromatasehemmer vor dem N-Bisphosphonat verabreicht wird.

11. Verwendung nach Anspruch 5 bei einem Patienten, der unter einer östrogenabhängigen Störung leidet.

12. Verwendung nach Anspruch 5 bei einer postmenopausalen Frau mit ER+ und/oder PR+ Brustkrebs.

## Revendications

1. Utilisation (a) d'un inhibiteur de l'aromatase dans la préparation d'un médicament utilisable en combinaison avec (b) un N-bisphosphonate destiné au traitement d'une maladie ou d'un état qui répond à l'inhibition de l'aromatase à condition que ladite maladie ou ledit état ne soit pas également traité à l'aide d'un agent chimiothérapeutique.

2. Utilisation selon la revendication 1 **caractérisée en ce que** la maladie est une maladie proliférative.

3. Utilisation selon la revendication 1 ou 2 **caractérisé en ce que** le N-bisphosphonate est administré une fois tous les six mois.

4. Combinaison comprenant (a) un inhibiteur de l'aromatase et (b) un N-bisphosphonate **caractérisée en ce que** les ingrédients actifs (a) et (b) sont présents dans chacun des cas sous forme libre ou sous forme d'un sel pharmaceutiquement acceptable, en vue d'une utilisation simultanée, concurrente, séparée ou séquentielle destinée à prévenir la perte de substance osseuse qui est provoquée par le traitement à l'aide d'un inhibiteur de l'aromatase d'une maladie ou d'un état qui répond à l'inhibition de l'aromatase.

5. Utilisation (a) d'un inhibiteur de l'aromatase pour la préparation d'un médicament utilisable en combinaison avec (b) un N-bisphosphonate destiné à prévenir la perte de substance osseuse qui est provoquée par le traitement à l'aide d'un inhibiteur de l'aromatase d'une maladie ou d'un état qui répond à l'inhibition de l'aromatase.

6. Utilisation selon l'une quelconque des revendications 1 à 3 ou 5 ou combinaison selon la revendication 4 **caractérisée en ce que** (a) l'inhibiteur de l'aromatase est sélectionné à partir de l'exemestane, du formestane, de l'aminoglutéthimide, du vorozole, du fadrozole, de l'anastrozole, du rogléthimide, du pyridoglutéthimide, du trilostane, du testolactone, de l'atamestane, du 1-méthyl-1,4-androstadiène-3,17-dione, du kétokonazole et d'un composé de formule I 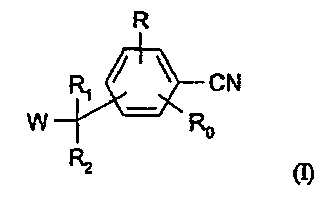 dans laquelle, R et R₀, indépendamment l'un de l'autre, sont chacun hydrogène ou alkyle inférieur, ou R et R₀ au niveau des carbones adjacents, de pair avec le cycle benzène auquel ils sont liés, forment un cycle naphtalène ou tétrahydronaphtalène ; dans laquelle R₁ est hydrogène, alkyle inférieur, aryle, aryl-alkyle inférieur ou alkényle inférieur ; dans laquelle R₂ est hydrogène, alkyle inférieur, aryle, aryl-alkyle inférieur, (alkyle inférieur, aryle ou aryl-alkyle inférieur)-thio ou alkényle inférieur, ou dans laquelle R₁ et R₂ ensemble sont alkylidène inférieur ou C₄-C₆alkylène ; dans laquelle W est 1-imidazolyle, 1-(1,2,4 ou 1,3,4)-triazolyle, 3-pyridyle ou un des radicaux hétérocycliques mentionnés ci-dessus substitués par alkyle inférieur ; et aryle, dans le cadre des définitions ci-dessus possède les significations suivantes : phényle qui est non substitué ou substitué par un ou deux substituants sélectionnés à partir du groupe composé d'alkyle inférieur, alcoxy inférieur, hydroxy, alkanoyloxy inférieur, nitro, amino, halogène, trifluorométhyle, cyano, carboxy, alcoxycarbonyle inférieur, carbamoyle, N-alkylcarbamoyle inférieur, N,N-di-alkylcarbamoyle inférieur, alkanoyle inférieur, benzoyle, alkylsulfonyle inférieur, sulfamoyle, N-alkylsulfamoyle inférieur et N,N-di-alkylsulfamoyle inférieur ; ainsi que thiényle, indolyle, pyridyle ou furyle, ou un de ces quatre radicaux hétérocycliques mono-substitués par alkyle inférieur, alcoxy inférieur, cyano ou halogène ; ou un de leurs sels pharmaceutiquement acceptables et dans laquelle, « inférieur » signifie jusqu'à 7 atomes de carbone.

7. Utilisation ou combinaison selon la revendication 6 **caractérisée en ce que** l'inhibiteur de l'aromatase (a) est 4-[α-(4-cyanophényl)-1-(1,2,4-triazolyl)méthyl]-benzonitrile (letrozole) ou un de ses sels pharmaceutiquement acceptables.

8. Utilisation ou combinaison selon la revendication 7 **caractérisée en ce que** le N-bisphosphonate (b) est un composé de formule I 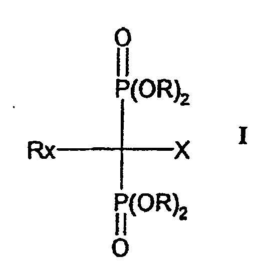 dans laquelle,
X est hydrogène, hydroxyle, amino, alkanoyle, ou un groupe aminé substitué par C₁-C₄alkyle, ou alkanoyle ;
R est hydrogène ou C₁-C₄alkyle et
Rx est une chaîne latérale qui contient un groupe aminé éventuellement substitué, ou un hétérocyclique contenant de l'azote (comprenant des hétérocycles contenant de l'azote aromatique), ou un de ses sels pharmaceutiquement acceptables ou n'importe lequel de ses hydrates.

9. Utilisation ou combinaison selon la revendication 8 dans laquelle le (b) N-bisphosphonate est un acide 2-(imidazol-1-yl)-1-hydroxyéthane-1,1-diphosphonique (acide zolédronique) ou un de ses sels pharmaceutiquement acceptables.

10. Utilisation selon l'une quelconque des revendications 1 à 3 ou 5 à 9 ou combinaison selon l'une quelconque des revendications 4 à 9 **caractérisée en ce que** l'inhibiteur d'aromatase (a) est administré préalablement au N-bisphosphonate.

11. Utilisation selon la revendication 5 auprès d'un patient souffrant d'un trouble dépendant de l' oestrogène.

12. Utilisation selon la revendication 5 auprès d'une femme post-ménopausée souffrant d'un cancer du sein ER+ et/ou PR+.
